# EUROPEAN PATENT APPLICATION

(11) **EP 4 015 623 A1**
(43) Date of publication of application: **22.06.2022**
(21) Application number: 20306596.6
(22) Date of filing: 17.12.2020
(51) Int. Cl.: C12N 5/0775, A61K 35/28, A61P 9/10

(54) **PRE-TREATMENT OF MSC WITH PPAR BETA/DELTA AGONIST FOR TREATMENT OF ISCHEMIA-REPERFUSION INJURY**

(71) Applicant: UNIVERSITE DE MONTPELLIER, 34090 Montpellier (FR); Centre National de la Recherche Scientifique (CNRS), 75016 Paris (FR); INSERM (Institut National de la Santé et de la Recherche Médicale), 75013 Paris (FR); Centre Hospitalier Universitaire de Montpellier, 34000 Montpellier (FR)
(72) Inventor: DJOUAD SAMRI, FARIDA, 34570 MONTARNAUD (FR); BARRERE-LEMAIRE, Stéphanie, 34680 SAINT GEORGES D'ORQUES (FR); JORGENSEN, CHRISTIAN, 34980 ST CLÉMENT DE RIVIÈRE (FR)
(74) Representative: Regimbeau

(57) **Abstract**

The present invention relates to an *in vitro* method for preparing a composition comprising mesenchymal stem cells (MSCs) intended for treating ischemia-reperfusion injury, comprising: a) providing MSCs; b) cultivating MSCs in a culture medium comprising a PPARβ/δ agonist; c) removing the culture medium and washing the MSCs; and d) collecting MSCs in a pharmaceutically acceptable carrier, wherein the pharmaceutically acceptable carrier does not contain a PPARβ/δ agonist. It further relates to MSCs obtained using the method of the invention, as well as their use as a medicament, preferably in the treatment or prevention of ischemia-reperfusion injury, wherein the method of treatment or prevention does not comprise administering a PPARβ/δ agonist to the subject.

## Description

### TECHNICAL FIELD OF THE INVENTION

The present invention is in the field of therapies intended for the treatment of ischemia-reperfusion injury based on the use of mesenchymal stem cells. It relates to an *in vitro* method for preparing a composition comprising mesenchymal stem cells (MSCs) intended for treating ischemia-reperfusion injury, comprising: a) providing MSCs; b) cultivating MSCs in a culture medium comprising a PPARβ/δ agonist; c) removing the culture medium and washing the MSCs; and d) collecting MSCs in a pharmaceutically acceptable carrier, wherein the pharmaceutically acceptable carrier does not contain a PPARβ/δ agonist. It further relates to MSCs obtained using the method of the invention, as well as their use as a medicament, preferably in the treatment or prevention of ischemia-reperfusion injury, wherein the method of treatment or prevention does not comprise administering a PPARβ/δ agonist to the subject.

### BACKGROUND ART

Myocardial reperfusion by revascularization of the culprit artery is the only treatment for acute myocardial infarction (AMI) patients. However, there is no treatment to specifically prevent irreversible ischemia-reperfusion (IR) injury, considered as the side effects of reperfusion mainly due to apoptosis and triggered by abrupt oxygen restoration in the ischemic tissue. Apoptosis is a highly regulated energy-dependent form of cell death of potential interest for therapeutic intervention because observed in ischemic disease both in animal models and in patients (Roubille, F. et al. Delayed postconditioning in the mouse heart in vivo. Circulation 124, 1330-1336, doi:CIRCULATIONAHA.111.031864 [pii] 10.1161/CIRCULATIONAHA.111.031864 (2011); ); Saraste, A. et al. Apoptosis in human acute myocardial infarction. Circulation 95, 320-323, doi:10.1161/01.cir.95.2.320 (1997)). This regulated mechanism of cell death is specific to the reperfusion phase. Indeed, apoptosis pre-activates during ischemia and is executed during reperfusion, due to its dependence on ATP production (McCully, J. D., Wakiyama, H., Hsieh, Y. J., Jones, M. & Levitsky, S. Differential contribution of necrosis and apoptosis in myocardial ischemia-reperfusion injury. Am J Physiol Heart Circ Physiol 286, H1923-1935, doi:10.1152/ajpheart.00935.2003 (2004)). Targeting specifically apoptotic cascades appears as a main strategy to prevent reperfusion injury and to limit infarct size (Boisguerin, P. et al. A novel therapeutic peptide targeting myocardial reperfusion injury. Cardiovasc Res 116, 633-644, doi:10.1093/cvr/cvz145 (2020)). Therefore, strategies targeting IR-induced apoptosis post-AMI need to be developed.

Mesenchymal stem cells (MSC) based therapy has been reported to improve the post-myocardial infarction (MI) functional recovery of the myocardium via a myriad of pathways that include the increase of endogenous cell survival, proliferation and angiogenesis. In addition, MSC potently repress inflammation and apoptosis through their plasticity and their capacity to release bioactive molecules and transfer organelles such as mitochondria and extracellular vesicles (EV) (Karantalis, V. & Hare, J. M. Use of mesenchymal stem cells for therapy of cardiac disease. Circ Res 116, 1413-1430, doi:10.1161/CIRCRESAHA.116.303614 (2015)). Based on these properties, MSC have been tested in several preclinical studies with promising results. Indeed, in preclinical studies of AMI, MSC injection improves tissue repair and cardiac function (Cashman, T. J., Gouon-Evans, V. & Costa, K. D. Mesenchymal stem cells for cardiac therapy: practical challenges and potential mechanisms. Stem Cell Rev Rep 9, 254-265, doi:10.1007/s12015-012-9375-6 (2013)), regulates the inflammatory response, decreases cell apoptosis (Tompkins, B. A. et al. Preclinical Studies of Stem Cell Therapy for Heart Disease. Circ Res 122, 1006-1020, doi:10.1161/CIRCRESAHA.117.312486 (2018)) and mortality in animals (Llano, R. et al. Intracoronary delivery of mesenchymal stem cells at high flow rates after myocardial infarction improves distal coronary blood flow and decreases mortality in pigs. Catheter Cardiovasc Interv 73, 251-257, doi:10.1002/ccd.21781 (2009)). In clinical trials, although the safety and efficacy of MSC in phase I and II have been demonstrated, inconsistencies in phase III trials have been reported (Jeong, H. et al. Mesenchymal Stem Cell Therapy for Ischemic Heart Disease: Systematic Review and Meta-analysis. International journal of stem cells, doi:10.15283/ijsc17061 (2018); Roncalli, J. et al. Intracoronary autologous mononucleated bone marrow cell infusion for acute myocardial infarction: results of the randomized multicenter BONAMI trial. European heart journal 32, 1748-1757, doi:10.1093/eurheartj/ehq455 (2011); Chen, Z., Chen, L., Zeng, C. & Wang, W. E. Functionally Improved Mesenchymal Stem Cells to Better Treat Myocardial Infarction. Stem Cells Int 2018, 7045245, doi:10.1155/2018/7045245 (2018)). These inconsistencies have been, in part, attributed to the poor *in vivo* survival rate and engraftment displayed by MSC but also to the source of MSC used and the deleterious effects of the *in vitro* amplification process of MSC functions (Chen, Z., Chen, L., Zeng, C. & Wang, W. E. Functionally Improved Mesenchymal Stem Cells to Better Treat Myocardial Infarction. Stem Cells Int 2018, 7045245, doi:10.1155/2018/7045245 (2018)). Therefore, to optimize MSC therapeutic potential and bring them into the routine clinical practice, it has been proposed that MSC need to be enhanced/augmented by increasing their survival rate and engraftment in the damaged tissue as well as their anti-apoptotic properties on cardiac cells.

Peroxisome proliferator-activated receptors (PPARs) are nuclear receptors that exist in three different isoforms: PPARα, PPARβ/δ and PPARγ. They heterodimerize with retinoid X receptor (RXR) and, after ligand binding, act as transcriptional regulators. Dependent on the tissue ligands and cofactors PPAR isoforms exert multiple functions (Wagner, K. D. & Wagner, N. Peroxisome proliferator-activated receptor beta/delta (PPARbeta/delta) acts as regulator of metabolism linked to multiple cellular functions. Pharmacology & therapeutics 125, 423-435, doi:10.1016/j.pharmthera.2009.12.001 (2010)). While PPARα is mainly expressed in brown adipose tissue, intestine, heart, liver, kidney, PPARγ is expressed in immune cells, gut, white and brown adipose tissue, and PPARβ/δ ubiquitously expressed. PPARβ/δ, a proangiogenic member of the PPAR family is expressed in vascular cell (Bishop-Bailey, D. Peroxisome proliferator-activated receptors in the cardiovascular system. Br J Pharmacol 129, 823-834, doi:10.1038/sj.bjp.0703149 (2000)). PPARβ/δ activation in HEK 293 cells deficient for cytoplasmic prostacyclin receptors is proapoptotic (Hatae, T., Wada, M., Yokoyama, C., Shimonishi, M. & Tanabe, T. Prostacyclin-dependent apoptosis mediated by PPAR delta. The Journal of biological chemistry 276, 46260-46267, doi:10.1074/jbc.M107180200 (2001)). Later studies reported that the antiapoptotic properties of PPARβ/δ in endothelial cells is mediated through the activation of endothelial 14-3-3 (Liou, J. Y., Lee, S., Ghelani, D., Matijevic-Aleksic, N. & Wu, K. K. Protection of endothelial survival by peroxisome proliferator-activated receptor-delta mediated 14-3-3 upregulation. Arteriosclerosis, thrombosis, and vascular biology 26, 1481-1487, doi:10.1161/01.ATV.0000223875.14120.93 (2006); Brunelli, L., Cieslik, K. A., Alcorn, J. L., Vatta, M. & Baldini, A. Peroxisome proliferator-activated receptor-delta upregulates 14-3-3 epsilon in human endothelial cells via CCAAT/enhancer binding protein-beta. Circ Res 100, e59-71, doi:10.1161/01.RES.0000260805.99076.22 (2007)). In line with these studies, the selective PPARδ agonist, GW501516, was shown to protect the cardiomyoblast cell line H9c2 from H₂O₂-induced cell death (Pesant, M. et al. Peroxisome proliferator-activated receptor delta (PPARdelta) activation protects H9c2 cardiomyoblasts from oxidative stress-induced apoptosis. Cardiovasc Res 69, 440-449, doi:10.1016/j.cardiores.2005.10.019 (2006)).

PPARβ/δ agonists have also been proposed in WO2015/164506 for use as stem cell engraftment enhancer in a combination therapy comprising co-administration of stem cell and PPARβ/δ agonists for treating various pathologies. In particular, while the main use disclosed in this application is the use of a combination of stem cells overexpressing prostacyclin (PGI2) and PGI2 for the treatment of a vascular-associated disease and/or a muscular disease, example 6 of this application discloses the combined use of MSC and PPARβ/δ agonist GW501516 in the context of ischemic hindlimbs. MSCs are pretreated by PPARβ/δ agonist GW501516 during 4 days in vitro and directly injected into the gastrocnemius of ischemic hindlimbs of NOD/SCID mice, which also receive daily administrations of PPARβ/δ agonist GW501516.

PPARβ/δ agonists have also been proposed for increasing cell transduction efficiency of a gene delivery vector (in particular a retroviral vector) in various cell populations (including MSCs, although experimental data is focused on hematopoietic stem cells - HSC) in WO2014/015318.

However, the in vivo administration of PPARβ/δ agonists may lead to significant deleterious effects. In particular, it was found that PPARβ/δ agonist GW501516 caused cancer to develop rapidly in several organs, at dosages of 3 mg/kg/day in both mice and rats (Sahebkar A, Chew GT, Watts GF (2014). "New peroxisome proliferator-activated receptor agonists: potential treatments for atherogenic dyslipidemia and non-alcoholic fatty liver disease". Expert Opin Pharmacother. 15 (4): 493-503; Geiger LE, Dunsford WS, Lewis DJ, Brennan C, Liu KC, Newsholme SJ (2009). PS 895 - Rat carcinogenicity study with GW501516, a PPAR delta agonist. 48th Annual Meeting of the Society of Toxicology. Baltimore: Society of Toxicology. p. 105; Newsholme SJ, Dunsford WS, Brodie T, Brennan C, Brown M, Geiger LE (2009). PS 896 - Mouse carcinogenicity study with GW501516, a PPAR delta agonist. 48th Annual Meeting of the Society of Toxicology. Baltimore: Society of Toxicology. p. 105).

There is thus a need for improved methods for treating ischemia-reperfusion injury by cellular therapy, in particular in the context of myocardial infarction, that would permit efficient engraftment of MSCs with antiapoptotic properties, without inducing deleterious effects of a co-administration of PPARβ/δ agonists as disclosed in the prior art.

### SUMMARY OF THE INVENTION

PPARβ/δ is highly expressed by MSC (Luz -Crawford, P. et al. PPARbeta/delta directs the therapeutic potential of mesenchymal stem cells in arthritis. Annals of the rheumatic diseases, doi:10.1136/annrheumdis-2015-208696 (2016)), but its role on MSC antiapoptotic and cardioprotective properties has not been investigated.

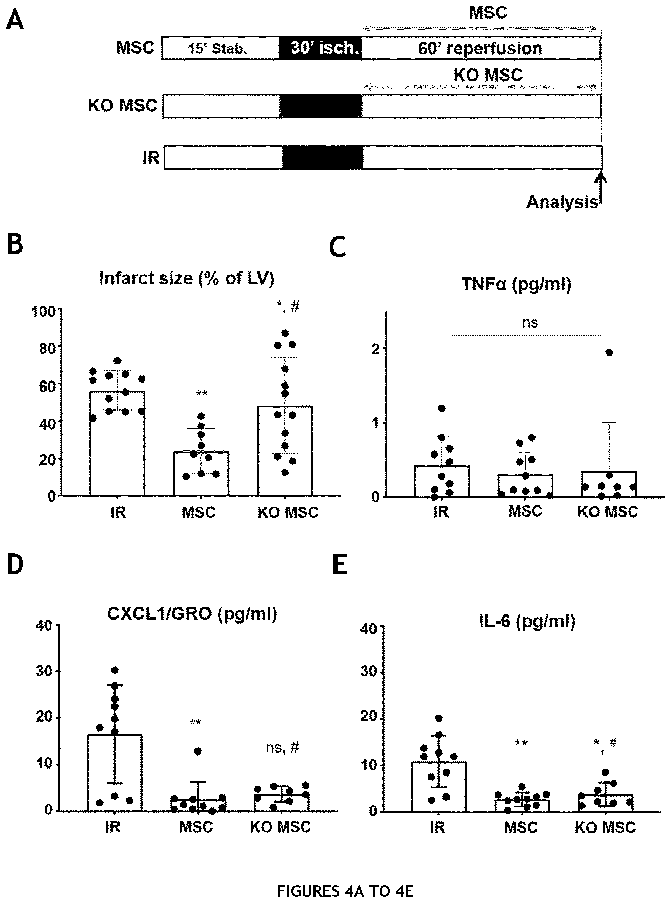

In the context of the present invention, the inventors found that the expression of PPARβ/δ by MSC was essential to their therapeutic efficiency in the treatment of myocardial ischemia-reperfusion injury. Indeed, while a knock down of PPARβ/δ did not alter their anti-inflammatory properties, it abrogated their cardioprotective effect (see Example 1, in particular Figure 4B), suggesting that the enhancement of MSC immunoregulatory properties to improve their beneficial effect in AMI should not be the only or main approach to overcome phase III clinical trial inconsistencies, but rather that it should be considered concomitantly with the enhancement of their survival once administrated to the patient.

Based on these first results, the inventors further surprisingly found that a short pretreatment (only 24 hours) of MSCs by a PPARβ/δ agonist is sufficient for significantly improving the survival, engraftment and anti-apoptotic properties of MSCs in the context of myocardial ischemia-reperfusion injury, leading to similar therapeutic efficiency at doses twice lower than when using non-pretreated MSCs. In this method, natural (non-transduced) MSCs were pretreated by the PPARβ/δ agonist, which was then washed away, and a composition comprising the pretreated MSCs but not comprising any PPARβ/δ agonist was administered to the subject in need thereof, thus preventing any deleterious effect associated to *in vivo* administration of a PPARβ/δ agonist. Unexpectedly, such a short pretreatment is sufficient to significantly improve the engraftment and anti-apoptotic properties of MSCs and their therapeutic efficiency in the treatment of ischemia-reperfusion injury (see Example 2).

In a first aspect, the present invention thus relates to an *in vitro* method for preparing a composition comprising mesenchymal stem cells (MSCs) intended for treating ischemia-reperfusion injury, comprising: a) providing MSCs; b) cultivating MSCs in a culture medium comprising a PPARβ/δ agonist; c) removing the culture medium and washing the MSCs; and d) collecting MSCs in a pharmaceutically acceptable carrier, wherein the pharmaceutically acceptable carrier does not contain a PPARβ/δ agonist.

The present invention also relates to a composition comprising mesenchymal stem cells (MSCs), wherein said composition has been obtained by the *in vitro* method according to the invention.

The present invention also relates to a composition according to the invention, for use as a medicament, preferably in a method of treatment of ischemia-reperfusion injury in a subject in need thereof, wherein the method of treatment does not comprise administering a PPARβ/δ agonist to the subject.

### DESCRIPTION OF THE FIGURES

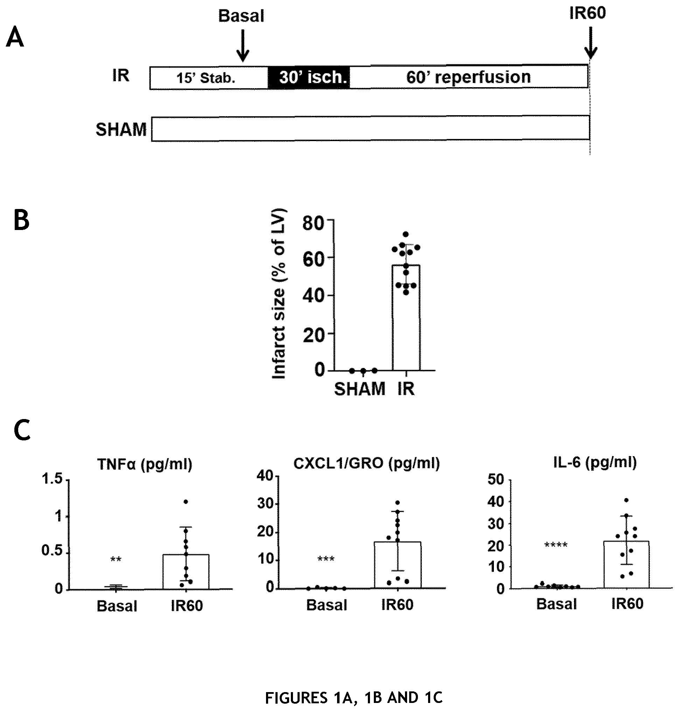

**Figure 1****:** C57Bl6 mouse hearts were mounted on a Langendorff system and subjected to IR injury. **(A):** The *ex vivo* protocol comprises a 15 minute-period of stabilization, followed by 30 minutes of global ischemia achieved by stopping the flow through the aorta (no-flow). Reperfusion was achieved by restoring the Tyrode perfusion during 60 minutes. For the SHAM condition, the heart was perfused during all the protocol without any ischemic induction. At the end of the protocol, infarct size was measured using the TTC-staining method. Coronary effluents were collected at two time points during the *ex vivo* protocol: during stabilization to evaluate the "basal" level of cytokine release and at the end of the reperfusion phase to evaluated the "IR60" cytokine production after IR injury. **(B):** Scatter plots and bars (mean ± SD) were represented for infarct size (in % of LV) in IR (n=12) and SHAM (n=3) hearts. Representative pictures of TTC-stained LV slices were shown for each group.
   (C): Scatter plots with bars (mean ± SD) are presented for quantification of cytokines within coronary effluents collected before ischemia (Basal) and after 60 min of reperfusion after an IR protocol (IR₆₀) using the Meso Scale Discovery (MSD) V-Plex Plus Proinflammatory Panel 1 (mouse) kit. Statistical analysis was performed using the Mann-Whitney test. For TNFα (pg/ml), ** was noted for p**=0.002, for CXCL1 (pg/ml), *** was noted for p***= 0.0007 and for IL-6 (pg/ml), ** was noted for p****<0.0001.

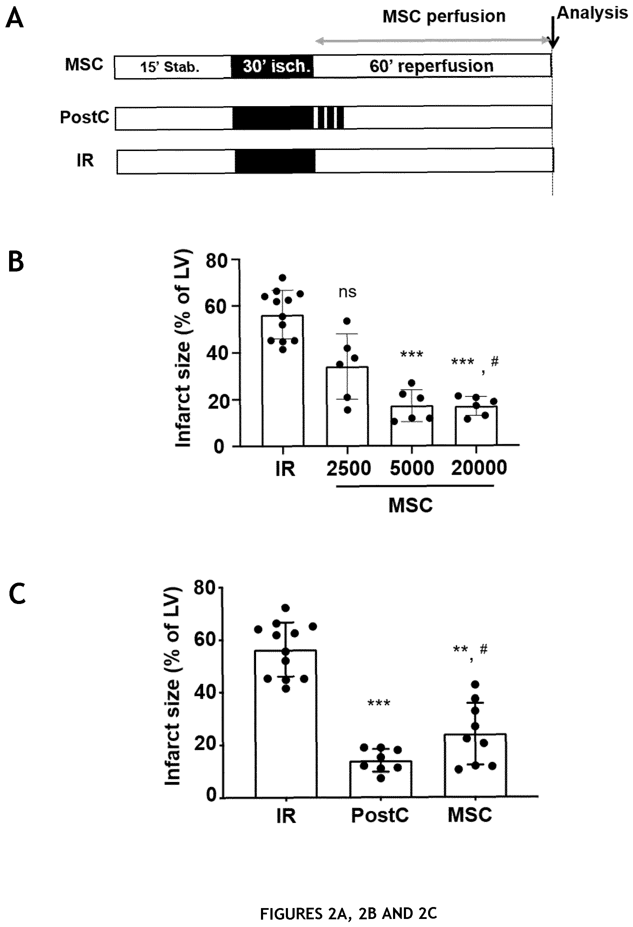

**Figure 2****.** Isolated hearts perfused *ex vivo* on the Langendorff system were submitted to perfusion protocol similar to that described in Figure 1A. **(A)** In the MSC group, reperfusion was achieved with a solution of MSC cells prepared in a Tyrode buffer at various concentrations (2,500; 5,000 or 20,000 cells/mL). For the PostC group, a postconditioning stimulus comprising 3 cycles of 1 min ischemia-1 min reperfusion was applied at the onset of reperfusion.
   In the control condition (IR), hearts were reperfused with Tyrode solution alone (control condition). Histological analysis was performed at the end of the protocols for infarct size measurement and immunochemistry.
   **(B):** Scatter plots and bars (mean ± SD) were represented for Infarct size (in % of LV) in IR (n=12), MSC 2500 cells/mL (n=6), MSC 5,000 cells/mL (n=6) and MSC 20,000 (n=6). Statistical analysis was performed using Kruskal-Wallis with the Dunn's post hoc test for multiple comparison. Statistical significance compared to IR is noted *** for p=0.0009, ns for p= 0.22 *vs* IR, # for p=0.84 vs MSC 5,000 cells/mL.
   **(C):** Scatter plots and bars (mean ± SD) were represented for Infarct size (in % of LV) in IR (n=12), PostC (n=6) and MSC (5,000 cells/mL, n=6). Statistical analysis was performed using Kruskal-Wallis with the Dunn's post hoc test for multiple comparison. Statistical significance compared to IR is noted *** for p=0.0002, ** for p=0.006 and # for p>0.99 vs PostC.

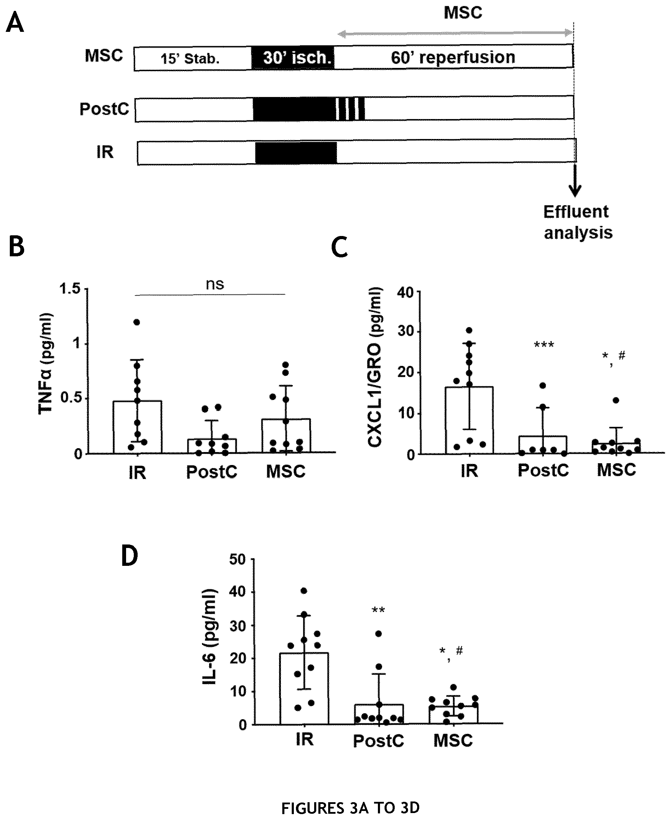

**Figure 3****. (A):** Isolated hearts perfused *ex vivo* on the Langendorff system were submitted to perfusion protocol similar to that described in Figure 1A. In the MSC group, reperfusion was achieved with a MSC Tyrode solution prepared at a concentration of 5000 cells/mL. For the PostC group, a postconditioning stimulus comprising 3 cycles of 1 min ischemia-1 min reperfusion was applied at the onset of reperfusion. In the control condition (IR), hearts were reperfused with Tyrode solution alone (control condition). Coronary effluents were collected at the end of the reperfusion phase to evaluate cytokine production after both IR injury and a PostC protocol.
   **(B, C, D):** Scatter plots with bars (mean ± **SD)** are presented for quantification of cytokines within coronary effluents collected after 60 min of reperfusion after an IR or a PostC protocol (IR and MSC, respectively) using the Meso Scale Discovery (MSD) V-Plex Plus Proinflammatory Panel 1 (mouse) kit. Statistical analysis was performed using the Kruskal-Wallis test. **(B):** For TNFα (pg/ml), ns was noted for p=0.108. **(C):** for CXCL1 (pg/mL), *** was noted for p***=0.0006 *(vs* IR), ** for p*=0.018 *(vs* IR) and ^{#}for p^{#}>0.999. **(D):** For IL-6 (pg/mL), ** was noted for p**=0.002 *(vs* IR), ** for p*=0.029 (vs IR) and ^{#}for p^{#}>0.999.
**Figure 4****. (A):** Isolated hearts perfused *ex vivo* on the Langendorff system were submitted to perfusion protocol similar to that described in Figure 1A. Reperfusion was achieved with a Tyrode solution alone in the IR group, in a Tyrode solution prepared at a concentration of 5000 cells/mL with MSC (MSC group) with KO MSC (KO MSC group). At the end of the protocol, infarct size analysis was performed on the isolated heart **(B)** and cytokine production was analyzed **(C, D, E)** using the Meso Scale Discovery (MSD) V-Plex Plus Proinflammatory Panel 1 (mouse) kit. Statistical analysis was performed using the Kruskal-Wallis test with the Dunn's post test when appropriate.
   **(B):** Scatter plots and bars (mean ± SD) were represented for Infarct size (in % of LV) in IR (n=12), MSC (5,000 cells/mL, n=9) and KO MSC (5,000 cells/mL, n=13). Statistical significance compared to IR is noted ** for p=0.001, * for p=0.029 and # for p=075 vs MSC. **(C, D, E):** Scatter plots with bars (mean ± **SD)** are presented for quantification of TNFα, CXCL1 and IL-6 within coronary effluents collected at 60 min after the onset of reperfusion from untreated hearts (IR), hearts treated with wild-type MSC (MSC) and MSC deficient for PPARβ/δ (KO MSC) using the Meso Scale Discovery kit. Statistical analysis was performed using the Kruskal-Wallis test. **(C):** For TNFα (pg/ml), ns was noted for p^{ns}=0.60. **(D):** for CXCL1 (pg/mL), ** was noted for p**=0.0017 (MSC vs IR), ns was noted for p=0.34 (KO MSC vs IR) and ^{#}for p^{#}=0.28 for (KO MSC vs MSC); **(E):** For IL-6 (pg/mL), ** was noted for p**=0.004 (MSC vs IR), * for p*=0.03 (KO MSC vs IR) and ^{#}for p^{#}>0.999 (KO MSC vs MSC).

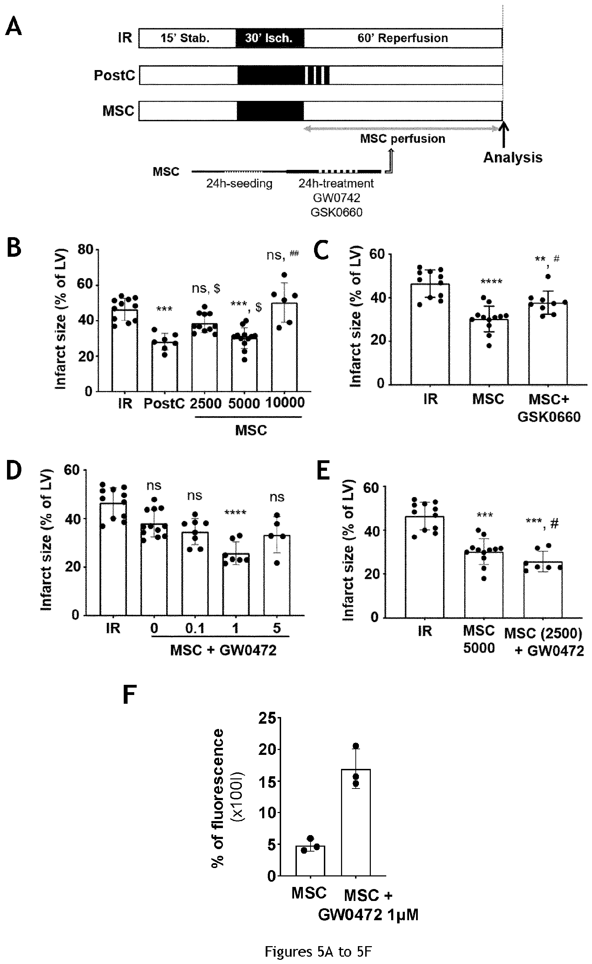

**Figure 5****: Increased cardioprotection in hearts treated by PPAR**β**/δ-primed MSC. (A)** C57Bl6 mouse hearts were mounted on a Langendorff system and subjected to IR injury. The *ex vivo* protocol comprises a 15 minute-period of stabilization, followed by 30 minutes of global ischemia achieved by stopping the flow through the aorta (no-flow). Reperfusion was achieved by restoring the Tyrode perfusion during 60 minutes (IR group). For the PostC group, a postconditioning stimulus comprising 3 cycles of 1 min ischemia-1 min reperfusion was applied at the onset of reperfusion. In the MSC group, reperfusion was achieved with a solution of MSC cells prepared in a Tyrode buffer at various concentrations (2,500; 5,000 or 10,000 cells/mL). At the end of the protocol, hearts were dedicated to infarct size (TTC-staining method; **B-E**) or immunochemistry (F) analysis. **(B)** Scatter plot and bars (mean ± SD) were represented for Infarct size in IR (n=11), PostC (n=7), MSC 2,500 cells/mL (n=11), MSC 5,000 cells/mL (n=10), and MSC 10,000 cells/mL (n=10); Statistical analysis was performed using Kruskal-Wallis with the Dunn's post hoc test for multiple comparison. Statistical significance compared to IR is noted *** for p=0.0004 (PostC vs IR), ns for p^{ns}= 0.80 (MSC 2500 *vs* IR), *** for p***=0.0004 (MSC 5,000 vs IR), ns for pns>0.99 (MSC 10,000 vs IR) and for comparisons vs PostC: $ for p$=0.097 (MSC 2,500), ^{$} for p^{$}>0.99 (MSC 5,000), ## for p^{##}= 0.001 (MSC 10,000). (C) Scatter plot and bars (mean ± SD) were represented for Infarct size in IR (n=11), MSC 5,000 cells/mL (n=12), MSC (5,000 cells/mL) + GSK0660 (n=9). Statistical analysis was performed by ANOVA (since the data passed the normality test) followed by the Tukey's multiple comparisons test. Statistical significance was note **** for p****<0.0001 *vs* IR, ** for p**=0.007 *vs* IR and # for p#=0.019 vs MSC. (D) Scatter plot and bars (mean ± SD) were represented for Infarct size in IR (n=11), MSC 2,500 cells/mL (n=12), MSC (2,500 cells/mL) + GW0742 0.1 µM (n=8), MSC (2,500 cells/mL) + GW0742 1 µM (n=7) and MSC (2,500 cells/mL) + GW0742 5 µM (n=5). Statistical analysis was performed by Kruskal-Wallis followed by the Dunn's post - test. Statistical significance was noted **** for p****<0.0001 (MSC + GW0742 1 µM *vs* IR), ns for p^{ns}=0.16 (MSC *vs* IR), ns for p^{ns}=0.056 (MSC + GW0742 0.1 µM *vs* IR) and ns for p^{ns}=0.0.07 (MSC+GW0742 5 µM *vs* IR). (E) Scatter plot and bars (mean ± SD) were represented for Infarct size in IR (n=11), MSC 5,000 cells/mL (n=12) and MSC (2,500 cells/mL) + GW0742 1 µM (n=7). Statistical analysis was performed by Kruskal-Wallis followed by the Dunn's post -test. Statistical significance was noted *** for p***=0.001 (MSC 5,000 *vs* IR), *** for p***=0.0003 (MSC 2,500+GW0742 vs IR) and ns for p^{ns}>0.99. (F) Scatter plot and bars (mean ± SD) for the % of fluorescence measured in the hearts injected with MSC (DI-I labelled MSC) *versus* MSC + GW0742 1 µM (n=3 for each group). Each dot represent the mean of the percentage of fluorescence obtained from n=17 to 26 slices of each heart.

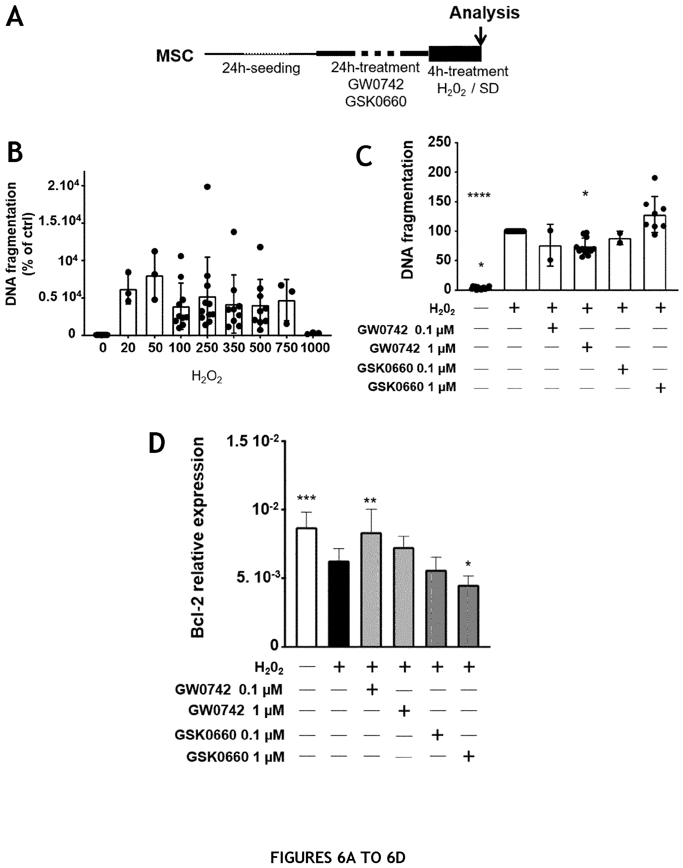

**Figure 6****: Increased resistance to oxidative stress and survival of PPAR**β**/δ-primed MSC. (A)** 48 hours after the seeding step in complete medium, MSC were subjected to H₂O₂-induced oxidative stress during 4 hours in minimal medium with serum deprivation. Pharmacological preconditioning of MSC using PPARβ/δ agonist or antagonist was performed 24 hours before oxidative stress exposure. At the end of the protocol, cell death or relative gene expression was analyzed measuring specific DNA fragmentation by ELISA and transcript amounts by RT-qPCR. **(B):** Scatter plot and bars were presented for specific DNA fragmentation quantification measured in MSC treated with H₂O₂ at various concentrations (0, 100, 150, 250, 500 and 750 µM) for 4 h in minimal medium. Data (normalized by the control value obtained with untreated MSC) were plotted as scatter dot blots and mean ± SD for each group of treatment and compared using Kruskal-Wallis (Dunn's *post hoc* test) and were noted **** for p<0.0001 (250 group, n=11 independent cultures vs 0 group, n=13), ** for p= 0.0013 (100 group, n= 10 *vs* 0 group, n=13), ** for p= 0.0015 (350 group, n= 9 *vs* 0 group, n=13), **for p= 0.0015 (500 group, n= 9 *vs* 0 group, n=13) and * for p= 0.0332 (750 group, n= 3 *vs* 0 group, n=13). **(C):** Scatter plots and bars were presented for specific DNA fragmentation quantification measured in MSC treated with 250 µM of H₂O₂ pretreated with GW0742 or GSK0660 at 0.1 and 1 µM 24 hours before H₂O₂ exposure. Data (normalized by the values obtained with H₂O₂-induced naive MSC) were plotted as scatter dot blots and mean ± SD for each group of treatment and compared using Kruskal-Wallis (Dunn's *post hoc* test). **P** values were noted **** for p<0.0001 (MSC, n=16 independent cultures vs MSC/H₂0₂, n=18), * for p= 0.0402 (MSC+1 µM GSK0660/H₂O₂, n= 8 vs MSC/H₂O₂, n=18), **(D)** Bcl-2 relative expression in MSC treated with H₂O₂ pretreated with GW0742 or GSK0660 at 0.1 and 1 µM 24 hours before H₂O₂ exposure.

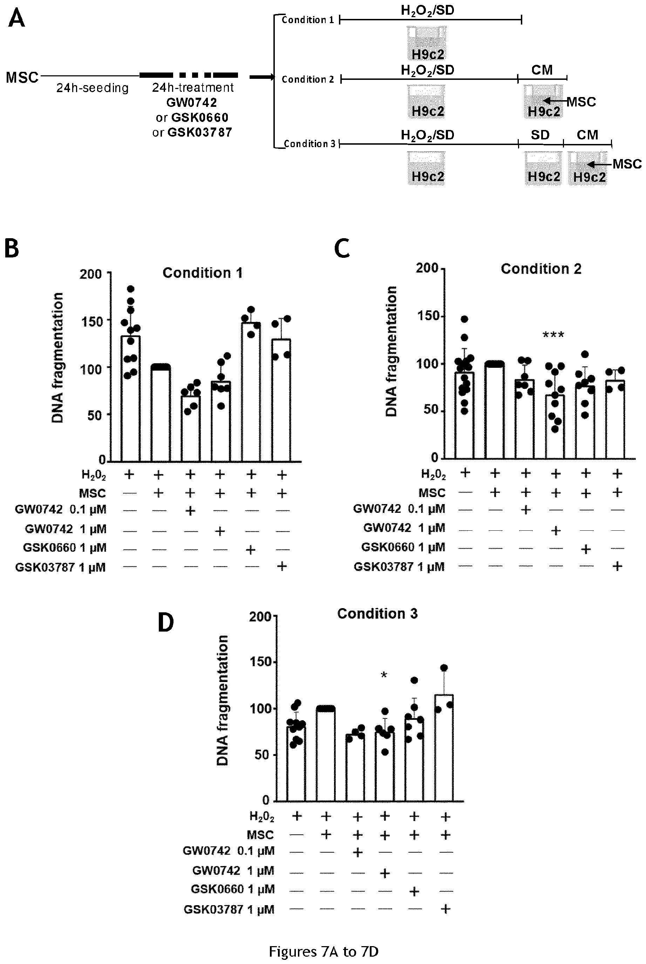

**Figure 7****: Increased anti-apoptotic effects of PPARβ/δ-primed MSC on cardiomyocytes. (A)** After 24 hours of MSC pharmacological preconditioning using PPARβ/δ agonist, cells were co-cultured using transwells (upper chamber) with H9c2 (lower chamber). Both cultures were exposed to 4 hours of 350 µM of H₂O₂ in minimal medium (serum deprivation). At the end of the protocol, specific DNA fragmentation was quantified in H9c2 cells and values were compared with those obtained for co-cultures with naive MSC or MSC pre-treated with PPARβ/δ antagonists (GSK0660 or GSK03787) and exposed to 350 H₂O₂ µM **(B, C, D)** Scatter dot plot and bars were presented for specific DNA fragmentation quantified in H9c2 at the end of the protocols. Data (mean ± SD for each group of treatment) were normalized by values obtained with naive MSC/H₂O₂ and compared using Kruskal-Wallis (Dunn's *post hoc* test). **(B):** data were obtained using the condition 1 protocol. **(C):** the condition 2 protocol P was followed and p value was noted **** for p<0.0001 (MSC, n=16 independent cultures *vs* MSC/ H₂0₂, n=18). For the data obtained with condition 3 protocol **(D),** p value was noted * for p= 0.0436 (MSC+1 µM GSK0660/H₂O₂, n= 6 *vs* MSC/H₂O₂, n=10).

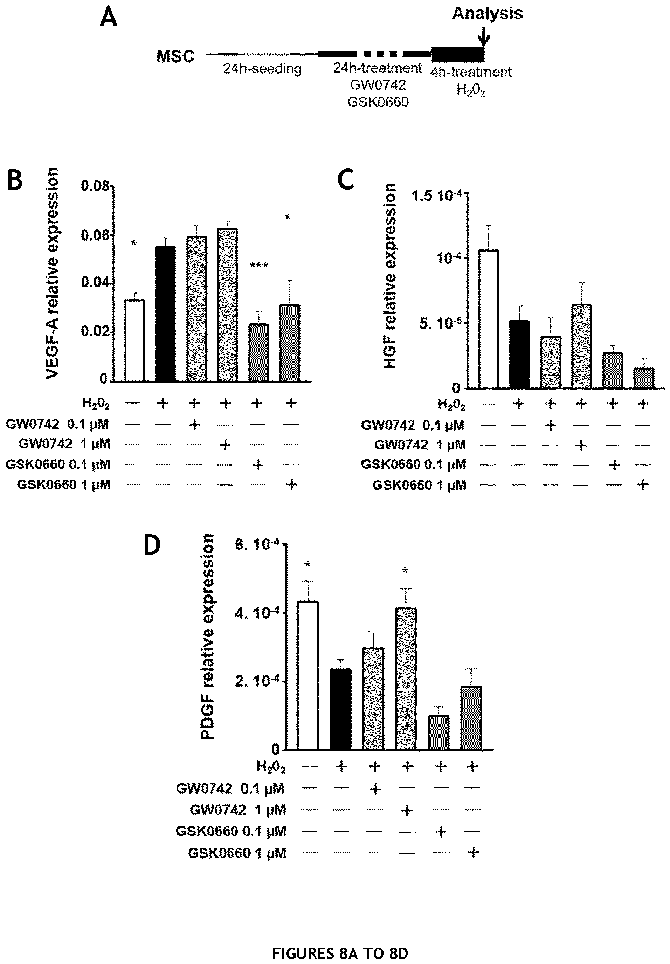

**Figure 8****: (A)** 48 hours after the seeding step in complete medium, MSC were subjected to H₂O₂-induced oxidative stress during 4 hours in minimal medium with serum deprivation. Pharmacological preconditioning of MSC using PPARβ/δ agonist or antagonist was performed 24 hours before oxidative stress exposure. At the end of the protocol, relative gene expression was analyzed measuring transcript amounts by RT-qPCR. mRNA expression levels in untreated or preconditioned MSC exposed to an oxidative stress were assessed by RT-qPCR for **(B)** *vegf:* data (mean ± SEM) were compared using ANOVA and the Tukey's post hoc for multiple comparisons (normality test passed). P values were noted for * for p= 0.0301 (control, n=9 *vs* H₂O₂, n=9)*** for p= 0.0009 (H₂O₂, n= 9 *vs* GSK0660 0.1 µM, n=9) and * for p=0.0191 (H₂O₂, n=9 *vs* GSK0660 1 µM, n=8); **(C)** for *hgf*: data (mean ± SEM) were compared using Kruskal-Wallis followed by the Dunn's *post-test* for multiple comparisons. P values were ns *versus* H₂O₂, n=7 for control n= 8, GWO742 0.1 µM n= 7, GW0742 1 µM n=9, GSK0660 0.1 µM n=6 and GSK0660 1 µM n=9; and **(D)** for *pdgf:* data (mean ± SEM) were compared using ANOVA and the Tukey's post hoc for multiple comparisons (normality test passed). P values were noted for * for p= 0.0132 (control, n=9 *vs* H₂O₂, n=9) and * for p= 0.0277 (H₂O₂, n= 9 *vs* GW07421 1 µM, n=9). For statistical analysis comparisons were made to the untreated MSC/H₂O₂ group.

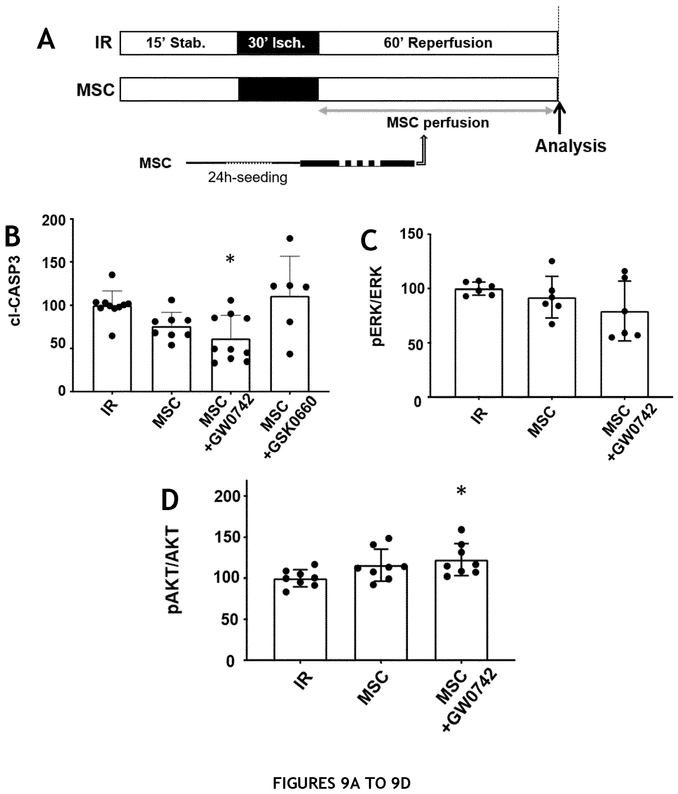

**Figure 9****: (A)** C57Bl6 mouse hearts were mounted on a Langendorff system. The *ex vivo* protocol comprises a 15 minute-period of stabilization, followed by 30 minutes of global ischemia achieved by stopping the flow through the aorta (no-flow). Reperfusion was achieved by restoring the Tyrode perfusion during 60 minutes (IR group). In the MSC group, reperfusion was achieved with a solution of MSC cells prepared in a Tyrode buffer (2500 cells/mL) pre-treated or not by GW0742 at 1 µM. At the end of the protocol, hearts were collected, protein extracted for further analysis. **(B, C, D)** *Western blot* analysis was performed from LV protein extracts from IR non-treated or MSC-treated murine IR hearts *(ex vivo)* with or without a pharmacological pre-treatment. Scatter dot blots and mean ± SD were plotted for Cleaved-caspase 3 (p*=0.0322 for IR, n=10 *vs* MSC +GW0742, n=10) and for pAKT/AKT (p*=0.0385 for IR, n=8 *vs* MSC +GW0742, n=8). Data were compared using non parametric Kruskal-Wallis test (Dunn's *post hoc* test).

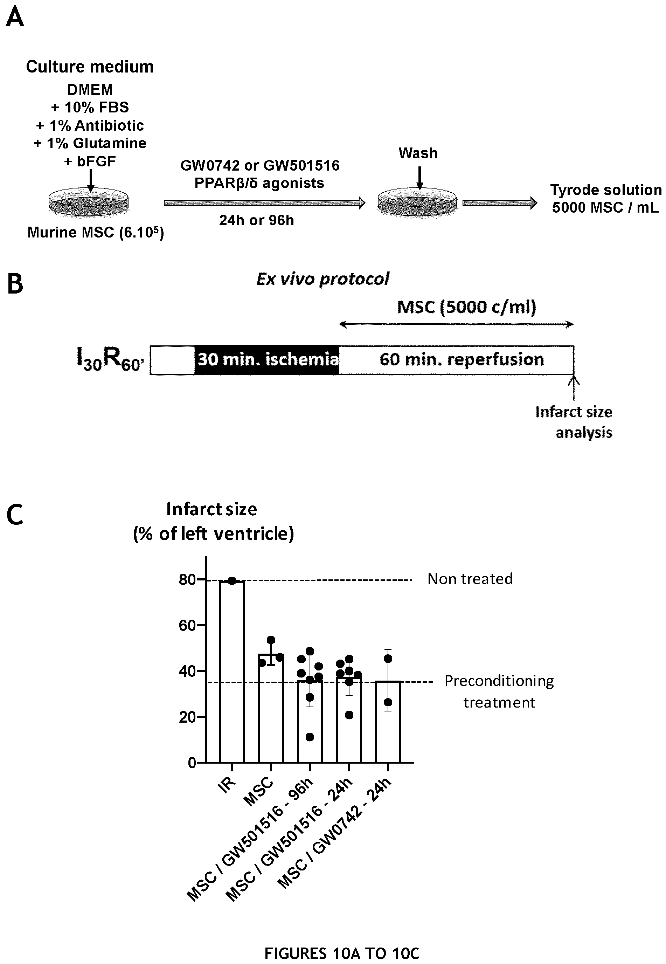

**Figure 10****: (A) MSC were cultured in a complete medium containing** in minimum essential medium (MEM)-α supplemented with 10% fetal bovine serum (FBS), 2 mM glutamine, 100 U/mL penicillin, 100 mg/mL streptomycin and 2 ng/ml human basic fibroblast growth factor (bFGF). 24 hours or 96h before the *ex vivo* experiment, MSC were incubated with a treatment solution containing the PPARβ/δ agonist diluted in the culture medium (GW0742, 1 µM and GW501516, 0.1 µM). The treatment lasted 24 hours or 96 hours. Cells were washed and after a trypsinization step, were centrifuged 3 min at 200g. MSC were re-suspended in a preheated Tyrode solution at the final concentration of 5000 cells/ml. **(B)** C56Bl6 mouse heart were perfused with a preheated Tyrode solution during 15 minutes (stabilization). Global ischemia was obtained by stopping the perfusion flow (« *no-flow* ») during 30 minutes. A reperfusion step (60 minutes) was achieved by restoring the flow with the MSC solution, which was applied during the 60 min-reperfusion step. The control condition (IR) was obtained using only Tyrode. **(C)** Scatter plot and bars (mean ± SD) were represented for Infarct size in IR (n=1; noted non treated on the graph), MSC (n=3), MSC + GW501516 0.1 µM (96h-preconditioning; n=8), MSC + GW501516 0.1 µM (24h-preconditioning; n=7) MSC + GW0742 1 µM (24h-preconditioning; n=2). MSC exhibit a similar cardioprotective effect whatever the preconditioning treatment and its duration.

### DETAILED DESCRIPTION OF THE INVENTION

### In vitro method for preparing a composition comprising mesenchymal stem cells (MSCs) intended for treating ischemia-reperfusion injury

The present invention first relates to an *in vitro* method for preparing a composition comprising mesenchymal stem cells (MSCs) intended for treating ischemia-reperfusion injury, comprising:
a) providing MSCs;
b) cultivating MSCs in a culture medium comprising a PPARβ/δ agonist;
c) removing the culture medium and washing the MSCs; and
d) collecting MSCs in a pharmaceutically acceptable carrier, wherein the pharmaceutically acceptable carrier does not contain a PPARβ/δ agonist.

### Step a)

In step a), mesenchymal stem cells are provided.

Minimal criteria to define human MSC have been defined in 2006 by the Mesenchymal and Tissue Stem Cell Committee of the International Society for Cellular Therapy and are still widely accepted today:
1. MSC must be plastic-adherent when maintained in standard culture conditions.
2. Phenotype: MSC must
   a. express (at least 95% of the cells) CD105 (known as endoglin and originally recognized by the MAb SH2), CD73 (known as ecto 5' nucleotidase and originally recognized by the MAb SH3 and SH4) and CD90 (also known as Thy-1), and
   b. lack expression (less than 2% of the cells) of CD45 (pan-leukocyte marker), CD34 (marker of primitive hematopoietic progenitors and endothelial cells), CD14 or CD11b (prominently expressed on monocytes and macrophages, the most likely hematopoietic cells to be found in an MSC culture, only one needs to be tested), CD79a or CD19 (markers of B cells, only one needs to be tested), and HLA-DR (not expressed on MSC unless stimulated) surface molecules.
3. In vitro differentiation: MSC must be able to differentiate to osteoblasts, adipocytes and chondroblasts in vitro.

### Tissue of origin of the MSCs

MSCs may be isolated from various tissues, including bone marrow (BM, the initial source of MSCs, Pittenger MF, Mackay AM, Beck SC, Jaiswal RK, Douglas R, Mosca JD, Moorman MA, Simonetti DW, Craig S, Marshak DR. Multilineage potential of adult human mesenchymal stem cells. Science 1999;284:143-147), adipose tissue (Wagner W, Wein F, Seckinger A, Frankhauser M, Wirkner U, Krause U, Blake J, Schwager C, Eckstein V, Ansorge W. Comparative characteristics of mesenchymal stem cells from human bone marrow, adipose tissue, and umbilical cord blood. Exp Hematol 2005;33:1402-1416; Zhang X, Yang M, Lin L, Chen P, Ma K, Zhou C, Ao Y. Runx2 overexpression enhances osteoblastic differentiation and mineralization in adipose-derived stem cells in vitro and in vivo. Calcif Tissue Int 2006;79:169-178), dental tissues (Huang GT, Gronthos S, Shi S. Mesenchymal stem cells derived from dental tissues vs. those from other sources: Their biology and role in regenerative medicine. J Dent Res 2009;88:792-806), salivary gland (Rotter N, Oder J, Schlenke P, Lindner U, Bohrnsen F, Kramer J, Rohwedel J, Huss R, € Brandau S, Wollenberg B. Isolation and characterization of adult stem cells from human salivary glands. Stem Cells Dev 2008;17:509-518), perinatal tissues such as placenta, umbilical cord tissue (Wharton's Jelly) or amniotic fluid (Raynaud C, Maleki M, Lis R, Ahmed B, Al-Azwani I, Malek J, Safadi F, Rafii A. Comprehensive characterization of mesenchymal stem cells from human placenta and fetal membrane and their response to osteoactivin stimulation. Stem Cells Int 2012; 2012:658356; Wang HS, Hung SC, Peng ST, Huang CC, Wei HM, Guo YJ, Fu YS, Lai MC, Chen CC. Mesenchymal stem cells in the Wharton's jelly of the human umbilical cord. Stem Cells 2004;22:1330-1337; Hou T, Xu J, Wu X, Xie Z, Luo F, Zhang Z, Zeng L. Umbilical cord Wharton's Jelly: a new potential cell source of mesenchymal stromal cells for bone tissue engineering. Tissue Eng Part A 2009;15:2325-2334; Kita K, Gauglitz GG, Phan TT, Herndon DN, Jeschke MG. Isolation and characterization of mesenchymal stem cells from the sub-amniotic human umbilical cord lining membrane. Stem Cells Dev 2010;19:491-502), menstrual blood (Luz-Crawford, P. et al. Mesenchymal Stem Cell-Derived Interleukin 1 Receptor Antagonist Promotes Macrophage Polarization and Inhibits B Cell Differentiation. Stem cells 2016; 34, 483-492, doi:10.1002/stem.2254; Francisca Alcayaga-Miranda et al.,Characterization of menstrual stem cells: angiogenic effect, migration and hematopoietic stem cell support in comparison with bone marrow mesenchymal stem cells. Stem Cell Res Ther 2015 Mar 17;6(1):32), peripheral blood (Weiping Lin et al., Characterisation of multipotent stem cells from human peripheral blood using an improved protocol. J Orthop Translat 2019; 7;19:18-28) and synovial tissue (Djouad, F., Bony, C., Häupl, T. et al. Transcriptional profiles discriminate bone marrow-derived and synovium-derived mesenchymal stem cells. Arthritis Res Ther 7, R1304 (2005); De Bari C, Dell'Accio F, et al. Multipotent mesenchymal stem cells from adult human synovial membrane. Arthritis Rheum. 2001 Aug;44(8):1928-42).

MSCs provided in step a) may thus be derived from bone marrow, adipose tissue, dental tissue, salivary gland derived, or perinatal tissue (such as placenta, umbilical cord tissue (Wharton's Jelly), amniotic fluid, menstrual blood, peripheral blood or synovial tissue. In a preferred embodiment, MSCs provided in step a) are bone marrow derived or adipose tissue derived MSCs, and in particular bone marrow derived MSCs.

### Subject of origin of the MSCs

MSCs provided in step a) are intended to be pretreated before their administration for treating ischemia-reperfusion injury. They may thus be either autologous (i.e. they are derived from a tissue of the subject to be treated) or allogeneic (i.e. they are derived from a tissue of another subject).

In the context of the invention, it is preferred that the MSCs provided in step a) are allogeneic and have been previously obtained from a healthy subject.

This permits to overcome individual variability and makes the preparation of the MSCs before administration to the subject suffering from ischemia-reperfusion injury simpler and much more rapid.

The MSCs provided in step a) may be obtained from a healthy subject just before step a). However, with the goal to limit individual variability and to start the method of preparation of the invention as soon as possible, the use of MSCs taken from a bank of previously isolated allogeneic MSCs derived from healthy subjects is preferred. In this embodiment, a bank of frozen samples of isolated allogeneic MSCs derived from healthy subjects has been previously generated and is used as a source of MSCs in step a).

### Isolation of MSCs

Methods for the isolation of MSCs from the various tissues mentioned above are known in the art. They generally rely on fresh samples of bone marrow and adipose tissue harvested on donors during surgical procedures.

When starting from bone marrow (BM), a suitable protocol may be as follows (Djouad, F., Bony, C., Häupl, T. et al. Transcriptional profiles discriminate bone marrow-derived and synovium-derived mesenchymal stem cells. Arthritis Res Ther 7, R1304 (2005)):
BM aspirates are resuspended in medium and centrifuged at 150 to 300 × *g* (preferably about 200 x g) for 5 to 15 minutes (preferably about 10 minutes) at ambient temperature. Cells are then plated at a density of 4 × 10⁴ to 6 × 10⁴ cells/cm² (preferably about 5 × 10⁴ cells/cm²) in α-MEM, supplemented with 5 to 15 % (preferably about 10%) fetal bovine serum, 1 to 10 ng/mL (preferably 1 ng/ml) basic fibroblast growth factor, antibiotics (usually penicillin and streptomycin). At confluency, cells are detached and subsequently replated at a density of 500 to 1,500 cells/cm² (preferably about 1,000 cells/cm²). Adherent cells between passage 2 and passage 7 constitute isolated BM-derived MSCs. When isolating human MSCs from human BM, a preferred protocol is disclosed in Djouad, F., Bony, C., Häupl, T. et al. Transcriptional profiles discriminate bone marrow-derived and synovium-derived mesenchymal stem cells. Arthritis Res Ther 7, R1304 (2005).

When starting from adipose tissue, a suitable protocol may be as follows (Maumus M, Manferdini C, et al. Adipose mesenchymal stem cells protect chondrocytes from degeneration associated with osteoarthritis. Stem Cell Res. 2013 Sep;11(2):834-44.): Adipose tissue is digested and centrifuged (at 150 to 300 x g, preferably about 300 x *g,* for 5 to 15 minutes, preferably about 10 minutes). The stroma vascular fraction is collected and seeded at an initial density of 3000 to 600 cell/cm² (preferably about 4000 cell/cm²) in α MEM supplemented with antibiotics (usually penicillin and streptomycin), glutamine and 5 to 15% (preferably about 10%) fetal calf serum (FCS). After 24 h to 48h (preferably about 24 hours), cultures are washed twice with a saline solution (preferably PBS). After 5 to 10 days (preferably about 1 week), cells are detached and expanded at 1000 to 3000 cells/cm² (preferably about 2000 cells/cm²) until they reach subconfluency.

Suitable protocols for isolating MSCs from dental tissues (Huang GT, Gronthos S, Shi S. Mesenchymal stem cells derived from dental tissues vs. those from other sources: Their biology and role in regenerative medicine. J Dent Res 2009;88:792-806), salivary gland (Rotter N, Oder J, Schlenke P, Lindner U, Bohrnsen F, Kramer J, Rohwedel J, Huss R, € Brandau S, Wollenberg B. Isolation and characterization of adult stem cells from human salivary glands. Stem Cells Dev 2008;17:509-518), and perinatal tissues such as placenta, umbilical cord tissue (Wharton's Jelly) or amniotic fluid (Raynaud C, Maleki M, Lis R, Ahmed B, Al-Azwani I, Malek J, Safadi F, Rafii A. Comprehensive characterization of mesenchymal stem cells from human placenta and fetal membrane and their response to osteoactivin stimulation. Stem Cells Int 2012; 2012:658356; Wang HS, Hung SC, Peng ST, Huang CC, Wei HM, Guo YJ, Fu YS, Lai MC, Chen CC. Mesenchymal stem cells in the Wharton's jelly of the human umbilical cord. Stem Cells 2004;22:1330-1337; Hou T, Xu J, Wu X, Xie Z, Luo F, Zhang Z, Zeng L. Umbilical cord Wharton's Jelly: a new potential cell source of mesenchymal stromal cells for bone tissue engineering. Tissue Eng Part A 2009;15:2325-2334; Kita K, Gauglitz GG, Phan TT, Herndon DN, Jeschke MG. Isolation and characterization of mesenchymal stem cells from the sub-amniotic human umbilical cord lining membrane. Stem Cells Dev 2010;19:491-502), are disclosed in the art.

### Step b)

In step b), MSCs are cultivated in a culture medium comprising a PPARβ/δ agonist.

### Seeding of MSCs

MSCs provided in step a) are seeded in a culture vessel comprising a culture medium. Since MSCs are adherent cells, they will preferably be seeded in a culture vessel suitable for the culture of adherent cells, such as 75 cm² flasks provided by Corning or Falcon suppliers.

A suitable seeding density for MSCs is comprised between 60 and 80% of confluency, i.e. between 3000 and 10000 cells/cm², preferably between 3000 and 7000 cells/cm², between 4000 and 6000 cells/cm², more preferably about 5,000 cells/cm2 (Bouffi C, Bony C, et al. IL-6-dependent PGE2 secretion by mesenchymal stem cells inhibits local inflammation in experimental arthritis. PLoS One. 2010 Dec 7;5(12):e14247).

### Culture medium

The culture medium used in step b) may be any culture medium suitable for the maintenance of MSCs. Such media include minimum essential medium (MEM)-α supplemented with fetal bovine serum (FBS), glutamine, antibiotics (usually penicillin and streptomycin), and human basic fibroblast growth factor (bFGF).

In one embodiment, the culture medium of step b) may be a minimum essential medium (MEM)-α supplemented with 5-15% (preferably 10%) fetal bovine serum, 1 to 5 mM (preferably 2 mM) glutamine, 50 to 200 U/mL (preferably 100 U/mL) penicillin, 50 to 200 mg/mL (preferably 100 mg/mL) streptomycin and 1 to 5 ng/mL (preferably 2 ng/mL) human basic fibroblast growth factor (bFGF).

### PPARβ/δ agonist

Data presented in Examples 2 and 3 show that several distinct PPARβ/δ agonists have the ability to significantly improve cardioprotective properties of MSCs when used in a pretreatment protocol. In addition, since the method of preparation according to the invention eliminates the PPARβ/δ agonist in step c) through removal of the culture medium and washing of the PPARβ/δ agonist-treated MSCs, even PPARβ/δ agonists for which in vivo administration has been shown to increase the risk of deleterious effects (such as cancer for PPARβ/δ agonist GW501516) may be used, since the PPARβ/δ agonist is not administrated to the subject.

Therefore, any PPARβ/δ agonist may be used in the method of preparation according to the invention.

Known PPARβ/δ agonists include the compounds presented in **Table 1** below, as disclosed in Takada I, Makishima M. Peroxisome proliferator-activated receptor agonists and antagonists: a patent review (2014-present). Expert Opin Ther Pat. 2020 Jan;30(1):1-13 (see in particular compounds 3 to 5 in Figure 1, and compounds 14 to 20 in Figure 3 of this article):

**Table 1. Known PPARβ/δ agonists that may be used in step b).**

| PPARβ/δ agonist name(s) | CAS number | Formula |
|---|---|---|
| GW0742 (also known as GW610742) | 317318-84-6 | 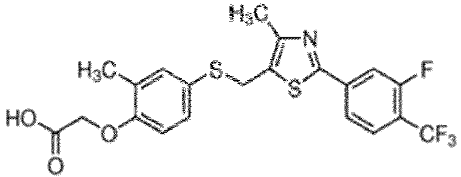 |
| GW501516 (also known as GW-501,516, GW1516, GSK-516, Cardarine) | 317318-70-0 | 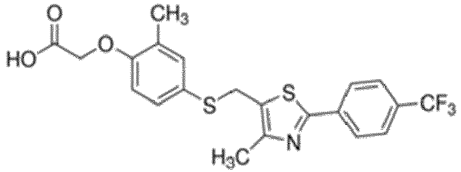 |
| L165041 or L-165041 | 79558-09-1 | 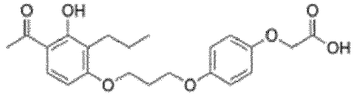 |
| MBX-8025 (also known as RWJ800025, RWJ-800025 and Seladelpar) | 851528-79-5 | 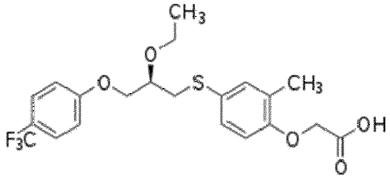 |
| KD-3010 | 934760-90-4 | 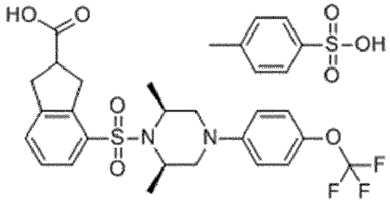 |
| vTv Therapeutics US9487493B2 | Unknown | 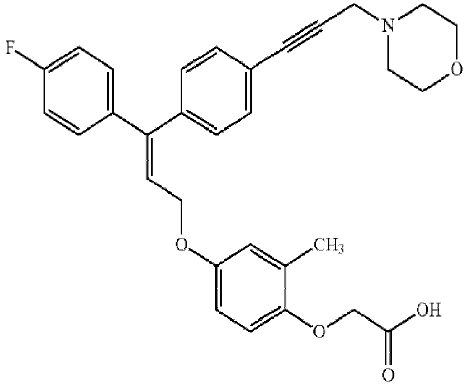 |
| University of Toledo US9695137B2 compounds 6a, 6b and 6c | Unknown | 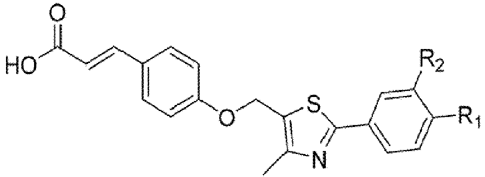 |
| | | 6a : R1 = R2 = H |
| | | 6b : R1 = CF₃, R2 = H |
| | | 6c : R1 = H, R2 = CF₃ |
| Mitobridge | Unknown | 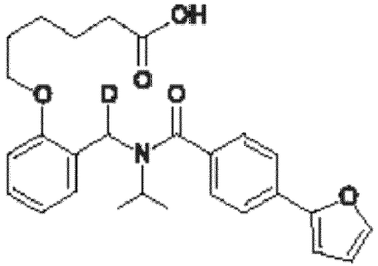 |
| Mitobridge WO2017180818A1 Compound 2i | Unknown | 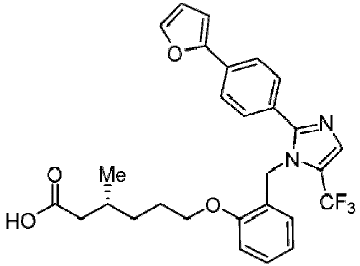 |
| Mitobridge WO2017180818A1 Compound 2c | Unknown | 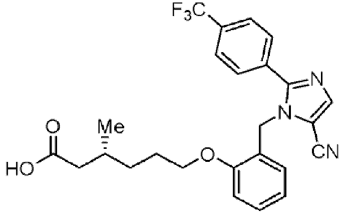 |

Further compounds proposed as agonists of PPARβ/δ that may be used in the context of the present invention are disclosed in US9487493B2, US9695137B2, US2017304255A1, US2018305403A1 and WO2017180818A1, all of which are herein incorporated by reference.

The PPARβ/δ agonist used in step b) may thus be selected from any PPARβ/δ agonist, in particular those disclosed in **Table 1** above, preferably from GW0742, GW501516, L165041, MBX-8025 and KD-3010. In a particularly preferred embodiment, the PPARβ/δ agonist used in step b) is selected from GW0742 and GW501516.

The concentration of PPARβ/δ agonist used in step b) will be adapted depending on the specific PPARβ/δ agonist chosen and its affinity for PPARβ/δ. However, based on data obtained in Examples 2 and 3, the concentration of the PPARβ/δ agonist (in particular compounds GW0742 and GW501516) in the culture medium of step b) is preferably comprised between 0.001 and 10 µM, more preferably between 0.01 µM and 10 µM, more preferably between 0.05 µM and 5 µM, and most preferably between 0.1 µM and 1 µM.

### Duration

Data presented in Examples 2 and 3 show that a short (24 hours) pretreatment of MSCs with a PPARβ/δ agonist is sufficient to significantly improve their cardioprotective properties.

Therefore, the duration of step b) is preferably comprised between 12 hours and 72 hours, preferably between 18 hours and 48 hours, more preferably between 20 hours and 30 hours, such as 24 hours.

### Absence of a viral vector for transducing the MSCs

Contrary to what is disclosed in WO2014/015318, MSCs prepared using the method of preparation according to the invention are preferably not genetically modified using transduction by a vector (in particular a viral vector, and notably a retroviral vector) expressing a heterologous gene.

Therefore, the culture medium of step b) preferably does not contain a viral vector for transducing MSCs.

### Step c)

In step c), the culture medium is removed and the MSCs are washed.

This step is very important because it permits to eliminate the PPARβ/δ agonist used for pretreating MSCs. As a result, the pre-treated MSC composition obtained using the method of preparation according to the invention does not contain or contains only trace levels of the PPARβ/δ agonist used in step b). This prevents any deleterious effect that might be associated to the in vivo administration of a PPARβ/δ agonist.

As MSCs are adherent cells, the culture medium may be easily removed by discarding or aspirating the culture medium.

The MSCs are then washed by adding a washing solution and removing it using similar methods as for the culture medium of step b). The washing solution does not comprise a PPARβ/δ agonist and is preferably selected from saline buffers (phosphate buffered saline, MEM). The step of washing (i.e. adding a washing solution and removing it) may optionally be repeated (i.e. more than one step of washing, such as 2 or 3 steps washing may be present), but only one step of washing will generally be sufficient to obtain a composition that does not comprise or comprises only traces of the PPARβ/δ agonist.

### Step d)

In step d), MSCs are collected in a pharmaceutically acceptable carrier, which does not comprise a PPARβ/δ agonist.

Said pharmaceutically acceptable carrier is a saline solution isotonic with blood, and may notably be selected from an isotonic solution of sodium chloride in water, phosphate-buffered saline (PBS) 1x and physiological serum. The following compounds may further be added to the saline solution isotonic with blood :
- Heparin, preferably at a concentration of 1 to 10 U/mL, since heparin was decribed in the rabbit model of IR injury to improve the efficacy of cell therapy (Ghadrdoost B, Khoshravesh R, et al. Heparin Enhances the Effects of Mesenchymal Stem Cell Transplantation in a Rabbit Model of Acute Myocardial Infarction. Niger J Physiol Sci. 2018 Jun 30;33(1):9-15), and/or
- human serum (Janssens S, Dubois C, et al. Autologous bone marrow-derived stem-cell transfer in patients with ST-segment elevation myocardial infarction: double-blind, randomised controlled trial. Lancet. 2006 Jan 14;367(9505):113-21) or human serum albumin (Chullikana A, Majumdar AS, Gottipamula S, et al. Randomized, double-blind, phase I/II study of intravenous allogeneic mesenchymal stromal cells in acute myocardial infarction. Cytotherapy. 2015 Mar;17(3):250-261), preferably at a concentration of 1 to 10 % (v/v).

As MSCs are adherent cells, their collection involves detaching them from the culture vessel. This may be done by exposing them to trypsin, trypsin-EDTA, or PBS-EDTA.

The volume of pharmaceutically acceptable carrier that is then used for collecting the MSCs is selected depending on:
- the total number of MSCs present in the culture vessel, and
- the desired final concentration of MSCs, which itself depends on the volume of liquid that is suitable for administration depending on the selected administration route.

For instance, when the pre-treated MSCs are intended for intravenous administration, a volume up to 150 mL (in particular from 30 to 150 mL) may be administered and a concentration of MSCs in the pharmaceutically acceptable carrier comprised between 10² and 10⁸ MSC cells/mL, preferably between 10³ and 10⁷ MSC cells/mL may be used, in particular in the treatment of myocardial ischemia-reperfusion injury.

When the pre-treated MSCs are intended for intracoronary administration, a volume up to 10 mL (in particular from 2 to 10 mL) may be administered and a concentration of MSCs in the pharmaceutically acceptable carrier comprised between 10⁶ and 10¹⁰ MSC cells/mL may be used, in particular in the treatment of myocardial ischemia-reperfusion injury.

### Optional freezing step e)

The method of preparation according to the invention may optionally further comprise a step e), in which the PPARβ/δ-primed MSCs are frozen for storage before use.

In this case, the PPARβ/δ-primed MSC composition is preferably frozen using specific MSC freezing solutions (notably Multiple Electrolytes Injection solutions such as Plasma-lyte A available from various suppliers, preferably containing cryopreservatives, such as human serum albumin (preferably 3-7%) and/or dimethyl sulfoxide (preferably 8-12%)) according to the supplier and stored at a temperature of -196°C in liquid nitrogen.

The frozen PPARβ/δ-primed MSC composition may be used to generate a bank of frozen PPARβ/δ-primed MSC samples.

### Composition comprising PPARβ/δ-primed MSCs obtained by the in vitro method of preparation according to the invention described above

The present invention also relates to a composition comprising PPARβ/δ-primed mesenchymal stem cells (MSCs), wherein said composition has been obtained by the *in vitro* method according to the invention.

"PPARβ/δ-primed MSCs" is herein intended to mean MSCs that have been treated by a PPARβ/δ-using the method according to the invention.

The PPARβ/δ-primed MSC composition according to the invention thus has the following features:
- It comprises MSCs shortly primed with a PPARβ/δ agonist, with improved cardioprotective properties,
- It does not comprise or comprises only traces of a PPARβ/δ agonist.

The PPARβ/δ-primed MSCs are in a pharmaceutically acceptable carrier, which does not comprise a PPARβ/δ agonist, and which is a saline solution isotonic with blood, and may notably be selected from an isotonic solution of sodium chloride in water, phosphate-buffered saline (PBS) 1x and physiological serum. The following compounds may further be present in the pharmaceutically acceptable carrier:
- Heparin, preferably at a concentration of 1 to 10 U/mL, since heparin was decribed in the rabbit model of IR injury to improve the efficacy of cell therapy (Ghadrdoost B, Khoshravesh R, et al. Heparin Enhances the Effects of Mesenchymal Stem Cell Transplantation in a Rabbit Model of Acute Myocardial Infarction. Niger J Physiol Sci. 2018 Jun 30;33(1):9-15), and/or
- human serum (Janssens S, Dubois C, et al. Autologous bone marrow-derived stem-cell transfer in patients with ST-segment elevation myocardial infarction: double-blind, randomised controlled trial. Lancet. 2006 Jan 14;367(9505):113-21) or human serum albumin (Chullikana A, Majumdar AS, Gottipamula S, et al. Randomized, double-blind, phase I/II study of intravenous allogeneic mesenchymal stromal cells in acute myocardial infarction. Cytotherapy. 2015 Mar;17(3):250-261), preferably at a concentration of 1 to 10% (v/v).

The composition according to the invention preferably contains pretreated MSCs in a therapeutically efficient amount. The inventors have found in a mouse model that reperfusion of a volume between 80 and 150 mL of a composition comprising pretreated MSCs at a concentration of 2.5 x 10³ MSC cells/mL, corresponding to a total number of PPARβ/δ-primed MSCs of 3.10⁵ cells, is as efficient as the double number of naïve (i.e. non-PPARβ/δ-primed) MSCs. The therapeutically efficient amount of PPARβ/δ-primed MSCs should thus be about half of the therapeutically efficient amount of naïve MSCs.

In humans, the numbers of naïve MSCs that have been found to show some efficiency in the treatment of ischemia-reperfusion injury in various contexts (MSC origin, administration route...) are comprised between 10⁷ and 10¹¹ cells. When using PPARβ/δ-primed MSCs, the therapeutically efficient amount should be lower, and may notably be comprised between 5x10⁶ and 5x10¹⁰ cells.

Depending on the volume that may be administered when using the selected administration route, a suitable volume and concentration of PPARβ/δ-primed MSCs will be selected for the PPARβ/δ-primed MSC composition according to the invention, as disclosed above in step d) of the method according to the invention.

As explained above in relation to step a) of the method of preparation according to the invention, while the MSCs of the composition may be either autologous or allogenic, they are preferably allogenic, and preferably obtained from a healthy subject.

The composition according to the invention is preferably a pharmaceutical composition.

### Therapeutic uses of the composition according to the invention

The inventors have unexpectedly shown that the composition according to the invention comprises PPARβ/δ-primed MSCs that display improved cardioprotective effects in case of myocardial ischemia-reperfusion injury, and is thus efficient the treatment or prevention of myocardial ischemia-reperfusion injury when administered alone, without concomitant administration of a PPARβ/δ agonist.

The present invention therefore also relates to a composition according to the invention, for use in a method of treatment or prevention, wherein the method of treatment or prevention does not comprise administering a PPARβ/δ agonist to the subject.

In particular, the present invention relates to a composition according to the invention, for use in a method of treatment or prevention of ischemia-reperfusion injury in a subject in need thereof, wherein the method of treatment or prevention does not comprise administering a PPARβ/δ agonist to the subject.

The present invention also relates to the use of a composition according to the invention for the manufacture of a medicament intended for the treatment or prevention of ischemia-reperfusion injury, wherein the treatment or prevention does not comprise administering a PPARβ/δ agonist to the subject.

The present invention also relates to the use of a composition according to the invention for the treatment or prevention of ischemia-reperfusion injury, wherein the treatment or prevention does not comprise administering a PPARβ/δ agonist to the subject.

The present invention also relates to a method for treating or preventing ischemia-reperfusion injury in a subject in need thereof, comprising administering to the subject a therapeutically efficient amount of the composition according to the invention.

By "treatment" or "treating" is meant an improvement of clinical or biological criteria in the subject when the composition according to the invention is administered after ischemia-reperfusion injury has occurred. For instance, "treatment" or "treating" may correspond to a decrease in apoptosis and/or inflammation in the organ of interest compared to before administration of the composition according to the invention.

By "prevention" or "preventing" is meant the fact to prevent or delay the onset or reduce the intensity of clinical or biological criteria associated to ischemia-reperfusion injury, when the composition according to the invention is administered after ischemia has occurred but before or during reperfusion of the organ of interest, i.e. before ischemia-reperfusion injury has occurred. For instance, "prevention" or "preventing" may correspond to the absence or weak occurrence of inflammation and/or apoptosis in the organ of interest following reperfusion of the organ, compared to what is generally observed in similar cases of reperfusion.

A "therapeutically effective amount" corresponds to an amount necessary to impart therapeutic or preventive benefit to a subject, as defined above.

### Ischemia-reperfusion injury

"Ischemia-reperfusion injury" or "IR injury" or "IRI" may be defined as the paradoxical exacerbation of cellular dysfunction and death, following restoration of blood flow to previously ischaemic tissues. While reestablishment of blood flow is essential to salvage ischaemic tissues, reperfusion itself paradoxically causes further damage, threatening function and viability of the organ. IRI occurs in a wide range of organs including the heart, lung, kidney, gut, skeletal muscle and brain and may involve not only the ischaemic organ itself but may also induce systemic damage to distant organs, potentially leading to multi-system organ failure.

The composition according to the invention may thus notably be used in the treatment of heart, lung, kidney, gut, skeletal muscle or brain ischemia-reperfusion injury.

Based on the data obtained by the inventors in myocardial ischemia-reperfusion injury, it will preferably be used in heart (and especially myocardial) ischemia-reperfusion injury.

Another case where ischemia-reperfusion injury is a significant problem is during organ transplantation (such as kidney transplantation), and the composition according to the invention may thus also be preferably used in the treatment of ischemia-reperfusion injury after organ transplantation, in particular after kidney transplantation.

### Administration schedules

Any suitable administration schedule may be used.

### Administration routes

In particular several administration routes may be used, depending on the type of ischemia-reperfusion injury treated.

In the case of heart (in particular myocardial) ischemia-reperfusion injury, suitable administration routes include intracoronary (via endovascular means), intravenous and intramyocardial (via open heart surgery means) or transendocardial (via endovascular means).

Intracoronary administration using endovascular means is particularly preferred because it may be done during coronarography, either at the time of reperfusion or later, coronarography being a relatively soft intervention that is regularly performed in patients that have suffered from a myocardial infarction.

Intravenous administration is less preferred because systemic administration means that the administered PPARβ/δ-primed MSCs will disseminate in various organs and many of them may be trapped in non-targeted organs (such as the lungs). However, intravenous administration has been used in patients after myocardial infarction in some clinical protocols (see Kobayashi K, Suzuki K. Mesenchymal Stem/Stromal Cell-Based Therapy for Heart Failure - What Is the Best Source? Circ J. 2018 Aug 24;82(9):2222-2232) using naïve MSCs and may thus also be used in the context of the present invention.

Intramyocardial administration has also been used in patients after myocardial infarction in some clinical protocols (see Kobayashi K, Suzuki K. Mesenchymal Stem/Stromal Cell-Based Therapy for Heart Failure - What Is the Best Source? Circ J. 2018 Aug 24;82(9):2222-2232) using naïve MSCs and may thus also be used in the context of the present invention. While intramyocardial administration may be performed either in the context of open-heart surgery or using endovascular means, intramyocardial administration using endovascular means is preferred.

In the case of ischemia-reperfusion injury after organ (notably kidney) transplantation, suitable administration routes include administration via endovascular means to the closest artery (intrarenal artery in the case of kidney graft).

### MSC dose

A therapeutically efficient dose of the PPARβ/δ-primed MSC composition according to the invention is used.

As explained above in the section relating to the PPARβ/δ-primed MSC composition according to the invention, the range of a therapeutically efficient dose will depend on various parameters, including the route of administration. However, a suitable dose range is between 5x10⁶ and 5x10¹⁰ PPARβ/δ-primed MSCs, in particular when administered by intracoronary administration.

### Timing of the administration

In the case of ischemia-reperfusion injury, a quick therapeutic intervention is critical. In particular, in AMI (acute myocardial infarction) patients, administration of MSCs should be performed within one week after AMI in order to be efficient.

Therefore, in a preferred embodiment, the PPARβ/δ-primed MSC composition according to the invention is administered within 1 week, preferably within 6 days, within 5 days, within 4 days, more preferably within 3 days, within 2 days or even within 24 hours of the onset of ischemia-reperfusion injury.

In another preferred embodiment, the PPARβ/δ-primed MSC composition according to the invention is administered during reperfusion of the ischemic organ, in order to prevent ischemia-reperfusion injury.

Such a quick response to ischemia or ischemia-reperfusion injury is made possible in the context of the invention by the short PPARβ/δ-priming of MSCs.

When using allogeneic MSCs, it is further made possible by the use of samples of a previously generated bank of MSCs. In one embodiment, the bank contains samples of naïve MSCs, ready to be PPARβ/δ-primed using the rapid method of preparation according to the invention, thus permitting the administration of the PPARβ/δ-primed MSCs within 2 days of the time the subject is taken in charge. In another embodiment, the bank contains samples of already PPARβ/δ-primed MSCs, permitting an administration within hours (such as within 24 hours, within 18 hours, within 12 hours, within 10 hours, within 8 hours, or even within 6 hours) of the time the subject is taken in charge.

The following examples merely intend to illustrate the present invention.

### EXAMPLES

### Example 1: PPARβ/δ is required for mesenchymal stem cell cardioprotective effects in myocardial ischemia-reperfusion injury

Myocardial infarction ranks first for the mortality worldwide. Since adult hearts are unable to regenerate, fibrosis expands to compensate the loss of contractile tissue after infarction, leading to cardiac remodeling and heart failure. Adult mesenchymal stem cells (MSC) regenerative properties, as well as their safety and efficacy have been demonstrated in preclinical models. However, in clinical trials, their beneficial effects are controversial. In an experimental model of arthritis, MSC derived from *PPARβ*/*δ* knockout (KO MSC) mice have been shown to provide a stronger therapeutic effect than MSC derived from wild-type mice.

The aim of this study was to compare the therapeutic effects of KO MSC cells and wild-type MSC *versus* untreated control condition (IR) against ischemia-reperfusion injury.

### Materials and Methods

### Animal housing and care

Experiments were carried out on C57BL/6J mice (Charles River laboratory) in accordance with the European Communities Council directive of November 1986 and conformed to the Guide for the Care and Use of Laboratory Animals" published by the US National Institutes of Health (NIH publication 8th Edition, 2011). All mice were maintained under environmentally controlled conditions (22 ± 2°C, 12 h light/12 h dark cycle) in the animal facility of the Institute. All *ex vivo* protocols were approved by the Ethical Committee for Animal Research (agreement #A34-172-41).

### Ex vivo experiments

Male mice were anesthetized with an intraperitoneal injection (IP) of ketamine (14 mg/kg, Imalgène^{®}; *Merial),* xylazine (14 mg/kg, Rompun^{®}; *Bayer)* followed by injection of pentobarbital (IP; 76.6 mg/kg; *Sanofi-Aventis).* Anesthetized mice received 250 U heparin (IP) in order to prevent blood clot formation. After sternotomy, the heart was excised, cannulated through the ascending aorta and quickly mounted on the Langendorff system. A pre-heated Tyrode solution (NaCl 140 mM, KCl 5.4 mM, MgCl 1 mM, Hepes 5 mM, glucose 5.5 mM, CaCl2 1.8 mM, pH 7.4) was perfused at constant pressure and temperature (37 °C).

### Ischemia-reperfusion protocol (IR)

On the Langendorff system, the heart was perfused with the preheated Tyrode solution during 15 minutes (stabilization). Global ischemia was obtained by stopping the perfusion flow (« *no-flow* ») during 30 minutes. A reperfusion step (60 minutes) was achieved by restoring the flow. The treatment with MSC was applied during reperfusion (MSC in Tyrode solution). The control condition (IR) was obtained using only Tyrode. Coronary effluents were collected all along the reperfusion protocol (at 5, 30 and 60 minutes post-reperfusion), and stored at -80°C for future experiments.

### Infarct size measurement

At the end of the IR protocol, the heart was dissected out. The left ventricle was included in agar (4%), and transversely sliced (1 mm) with a vibratome. To reveal tissue viability, slices were incubated with 2,3,5-triphenyltetrazolium chloride solution (TTC dye) at 37°C for 15 minutes. After a fixation step (4% paraformaldehyde, 48 h), each slice was photographed on each side. Infarct area was quantified by planimetric measurements with *Image J* software.

### MSC culture

Isolation, amplification and characterization of murine MSC was performed as previously described (Scholtysek, C. et al. PPARβ/δ governs Wnt signaling and bone turnover. Nat Med 19, 608-613, doi:10.1038/nm.3146 (2013)). Briefly, bone marrow (BM) was flushed out from long bones of Ppard fl/fl sox2cretg PPARβ/δ deficient mice referred as PPARβ/δ^{-/-} MSC and their wild-type Ppard fl/+ littermates referred as PPARβ/δ^{+/+} MSC provided by Béatrice Desvergne (Scholtysek, C. et al. PPARβ/δ governs Wnt signaling and bone turnover. Nat Med 19, 608-613, doi:10.1038/nm.3146 (2013)). Cells were cultured in minimum essential medium (MEM)-α containing 10% fetal bovine serum, 2 mM glutamine, 100 U/mL penicillin, 100 mg/mL streptomycin and 2 ng/ml human basic fibroblast growth factor (bFGF) at a density of 0.5×10⁶cells/cm². The phenotypic and functional characterization of MSC was previously performed (Luz-Crawford, P. et al. PPARbeta/delta directs the therapeutic potential of mesenchymal stem cells in arthritis. Annals of the rheumatic diseases, doi:10.1136/annrheumdis-2015-208696 (2016)). PPARβ/δ^{+/+} MSC were pre-incubated 24 hours with 5 µM of PPARβ/δ antagonist GSK0660.

### MSC labeling with CM-DiI.

Stock solution of the fluorescent cell-tracer CM-DiI (Molecular Probes) was reconstituted in dimethyl sulfoxide (DMSO) at a concentration of 1 µg/µL. MSC were collected and suspended at the concentration of 10⁷ cells/10 µg CM-DiI in 5 mL PBS. Cells were incubated at 37°C for 5 minutes followed by 15 minutes at 4°C, in the dark. Unincorporated fluorescent dye was then removed by centrifugation at 300 g for 5 minutes and 2 washes in PBS. Labeled cells were resuspended in PBS and maintained at 4°C prior to be injected in the myocardium.

### Cytokine quantification.

For cytokine quantification, the collect of effluents from the perfused hearts were obtained in basal conditions (t0), after 10 min stabilization, and at the end of the protocol after 60 min of reperfusion (IR60). The different effluents were stored at -80°C until the assay was performed using the Meso Scale Discovery (MSD) V-Plex Plus Proinflammatory Panel 1 (mouse) kit at the "Plateforme de Protéomique Clinique de Montpellier" according to the manufacturer's protocol. This panel included IFNγ, IL-1B, IL-2, IL-4, IL-5, IL-6, KC/GRO (CXCL1), IL-10, IL-12p70 and TNFα. Samples which were, for a particular cytokine, under the limit of detection i.e. with a concentration of cytokine undistinguishable from the background noise were considered negative for that cytokine.

### Immunochemistry

At the end of *ex vivo* experiments, left ventricles (LV) were fixated in 4%-PFA and embedded in paraffin. Each left ventricle was cut from the apex through the base (4 µm sections each 150 µm). Paraffin-embedded sections were deparaffinised then rehydrated through an alcohol gradient. LV sections were incubated with primary antibodies: anti-F4/80 (1:100; rat monoclonal; *Abcam*), anti CD68 Monoclonal Antibody (FA-11; *Invitrogen),* CX3CR1antibody (1H14L7; *Thermofischer Scientific)* and anti α-actinin (1:100, mouse monoclonal; *Sigma-Aldrich).* Cell nuclei were stained with Hoechst *(Life technologies* SAS) and endothelial cells with Isolectin B4 (FITC Conjugate; *Sigma-Aldrich).* After primary antibody incubation, the sections were washed in PBS, and incubated (3 hours) with secondary antibodies (1:2,000, *Jackson ImmunoRes Laboratories,* Inc.). Primary and secondary antibodies were diluted in PBS containing 3% BSA and 0.1% Triton X100. Stained sections were mounted in Mowiol (*Biovalley*). Images were obtained with a Zeiss Axioimager Z3 fluorescent microscope and analyzed using ImageJ and Adobe Photoshop to prepare final figures.

### Statistical analysis

Data expressed as mean ± SD values were compared using non-parametric Mann-Whitney (two groups) and Kruskal-Wallis (multiple comparison) using GraphPad Prism 8 Software. Graphs show mean ±Standard deviation (SD). P-values < 0.05 (*), P < 0.01 (**), P < 0.001 (***) and p<0.0001(****) were considered statistically significant. Analysis and graphical representation were performed using Graph-Pad Prism^{™} software (Graphpad).

### Results

### Induction of a pro-inflammatory response in isolated perfused heart subjected to ischemia-reperfusion injury ex vivo

For this study, we have developed an *ex vivo* model of global ischemia followed by reperfusion (IR protocol) to evaluate the therapeutic effects of MSC. Isolated hearts were mounted and perfused on a Langendorff system and subjected to 30 minutes of global ischemia (no-flow) followed by 60 minutes of reperfusion, phase during which the cells were administered (see protocol in **Figure 1A****).** The first part of the study was dedicated to the characterization of our model. Hearts after myocardial ischemia-reperfusion injury were characterized by a mean value infarct size of 56.4 ± 10.3 expressed in percentage of the left ventricle **(****Figure 1B****).** Immunohistology allowed to evidence resident macrophages in the myocardium that were located in the thickness of the myocardium (data not shown).

We thus wondered whether the induction of IR injury and the presence of macrophages in the myocardium were associated with an excessive release of pro-inflammatory cytokines in the coronary effluents collected at 60 minutes after the onset of reperfusion compared to the basal condition collected during stabilization (Basal). Among the ten cytokines quantified using the Meso Scale Discovery VPlex Plus Proinflammatory, seven including IFNγ, IL-1B, IL-2, IL-4, IL-5, IL-10, IL-12p70 were not included in statistical analyses because too many samples had undetectable levels. Three cytokines, TNFα, KC/GRO (CXCL1) and IL-6, showed highly significant increases in the coronary effluents of the hearts after IR injury (after ischemia and 60 min of reperfusion) as compared to their basal conditions (collected at 10 min stabilization, basal) **(****Figure 1C****).** Altogether, these results reveal that the myocardial IR injury of hearts from C57Bl6 mice subjected *ex vivo* to 30 minutes ischemia followed by 1-hour reperfusion is associated with the presence of macrophages that bathe in an inflamed myocardium.

### MSC exerted a potent cardioprotective effect when administered during reperfusion in an ex vivo model of global ischemia.

To reduce inflammation in the IR myocardium and thus infarct size, we relied on two well-admitted cardioprotective strategies that we compared for the first time in an *ex vivo* model: ischemic PostC and MSC-based therapy. To that end, MSC were administrated during reperfusion in isolated hearts subjected to 30 minutes of global ischemia as described in the protocol presented in **Figure 2A****.** Various concentrations of MSC in the perfusion solution were tested **(****Figure 2B****).** The dose-response curve was established using various doses of MSC and showed that 5,000 cells/mL (6.10⁵ cells/heart) and 20,000 cells/mL (24.10⁵ cells/heart) induced cardioprotective effects (17.29 ± 6,84, n=6 for MSC 5,000 vs 56.39 ± 10.33, n=12 for IR; p=0.0009 and 17.04 ± 4.07; n=6 for MSC 20,000 vs 56.39 ± 10.33, n=12; p=0.0009, respectively) by contrast to 2,500 cells/mL (3.10⁵ cells/heart) that did not provide a significant beneficial effect (34.1 ± 13.93, n=6 for MSC 2,500 *vs* 56.39 ± 10.33, n=12 for IR; p=0.2238). The concentration of 5,000 cells/mL was chosen for all the experiments of our study since it was the minimal concentration given the maximal cardioprotection in the *ex vivo* mouse model. Going further, we demonstrated that the reperfusion with MSC at 5,000 cells/mL (6.10⁵ cells/heart) decreased infarct size to the same extent as ischemic PostC, taken as positive control in our experiments (14.04 ± 4.32, n=8 for PostC *vs* 17.29 ± 6.84, n=6 for MSC, p>0.99; **Figure 2C****).** Immunohistological analysis revealed that MSC perfused *ex vivo* were located in the coronary microvessels after 1 hour of reperfusion (data not shown). Altogether, these results demonstrate that 6.10⁵ MSC /heart are as therapeutic as the ischemic PostC, the gold standard strategy in cardioprotection during AMI.

### The potent cardioprotective effect of both the ischemic PostC and MSC administration are associated with a decrease of proinflammatory cytokines within the injured myocardium

To determine whether the beneficial effect of the ischemic PostC and the administration of MSC on IR injury was associated with a regulation of the immune response, we quantified the proinflammatory cytokines within the coronary effluents collected at 15, 30 and 60 minutes after the onset of reperfusion **(****Figure 3A****).** Among the ten cytokines quantified using the MSD approach, seven including IFNγ, IL-1β, IL-2, IL-4, IL-5, IL-10, IL-12p70 were not detected in the samples. However, TNFα **(****Figure 3B****),** CXCL1 (KC/GRO) **(****Figure 3C****)** and IL-6 **(****Figure 3D****)** were present at significantly lower levels in the coronary effluents of the hearts subjected to an ischemic PostC or an administration of MSC. This result indicates that the protective effects of PostC and MSC were associated with a potent anti-inflammatory effect as assessed by the quantification of pro-inflammatory cytokines in the coronary effluents.

### PPAR β/δ is involved in the cardioprotective effects mediated by MSC against IR injury.

Recently, we have shown that PPARβ/δ is pivotal for MSC immunoregulatory and therapeutic functions in an experimental of arthritis (Luz-Crawford, P. et al. PPARbeta/delta directs the therapeutic potential of mesenchymal stem cells in arthritis. Annals of the Rheumatic Diseases 2016;75:2166-2174). However, the role of PPARβ/δ on MSC cardioprotective activity and the relevance of PPARβ/δ on MSC immunoregulatory properties in the inflamed myocardium have never been addressed. To determine whether PPARβ/δ is critical for MSC cardioprotective properties we compared the effect of MSC isolated from PPARβ/δ^{-/-} -deficient mice (KO MSC) and those obtained from their PPARβ/δ^{+/+} control littermates (MSC). Isolated hearts were perfused during reperfusion by solutions containing MSC at the optimal dose of 6.10⁵cells/heart (see protocol **Figure 4A****).** In these conditions, the drastic decrease in infarct size induced by wild-type MSC (24.1 ± 11.8, n=9 for MSC *vs* 56.4 ± 10.3, n=12 for IR; *p***=0.001) was abolished when KO MSC were perfused in the isolated hearts after the ischemic insult (48.4 ± 25.4, n=13 for KO MSC vs 56.4 ± 10.3, n=12 for IR; p^{ns} = 0.75 and 48.4 ± 25.4, n=13 for KO MSC vs 24.1 ± 11.8, n=9 for MSC; p*=0.029) **(****Figure 4B****).** To determine whether the loss of MSC therapeutic effect in response to PPARβ/δ knockdown was associated with a loss of their capacity to reduce inflammation in the infarcted myocardium, we quantified the pro-inflammatory cytokines within the coronary effluents collected at 60 minutes after the onset of reperfusion. MSD quantification of the cytokines did not show any change in TNFα concentration in the coronary effluents of MSC treated and untreated hearts. However, we demonstrated anti-inflammatory effects of MSC in hearts undergoing ischemic insults as revealed by the significant decrease in CXCL1 and IL-6 concentrations in the in coronary effluents collected at reperfusion (for CXCL1: 16.61 pg/ml ± 10.53, n=10 for IR *vs* 2.56 pg/ml ± 3.79, n=10; for MSC, p**=0.0017 **(****Figure 4D****)** and for IL-6: 21.83 pg/ml ± 11.08, n=10 for IR *vs* 5.43 pg/ml ± 3.02, n=10; for MSC, p**=0.004 **(****Figure 4E****)).** Of note, PPARβ/δ knockdown in MSC did not modify their anti-inflammatory potential as revealed by the concentrations of CXCL1 (2.56 pg/ml ± 3.79, n=10 for MSC *vs* 3.73 pg/ml ± 1.65, n=8 for KO MSC, p^{ns}=0.28; **Figure 4D****)** and IL-6 (5.43 pg/ml ± 3.02, n=10 for MSC vs 7.65 pg/ml ± 5.04, n=8 for KO MSC, p^{ns}>0.99; **Figure 4E****)** in coronary effluents collected at reperfusion that were maintained.

### Discussion and conclusions

After demonstrating that the *ex vivo* protocol of global ischemia (30 min) followed by 60 min-reperfusion used in that study induced a pro-inflammatory response as assessed by an increased level of TNFα, IL-6 and CXCL1 in coronary effluents collected at 60 min of reperfusion, we tested the role of PPARβ/δ on MSC immunoregulatory and therapeutic functions. First, we identified the optimal dose of MSC, 6. 10⁵cells/heart, allowing to provide a significant cardioprotection in the *ex vivo* mouse model with a minimal concentration of cells. Of note, at this dose of MSC chosen we observed a similar cardioprotection as the ischemic postconditioning (PostC) taken as positive control in our study. Indeed, both treatments allowed to reduce the pro-inflammatory response of IR injury and to decrease infarct size with the same efficacy. Moreover, this study reveals that MSC cardioprotective properties are PPARβ/δ-dependent by contrast to their anti-inflammatory effects on TNFα, CXCL1 and IL-6 release in coronary effluents.

Despite benefical effects in AMI patients, reperfusion therapy induces both local and systemic inflammation, called sterile inflammation that will reinforce the initial inflammatory response triggered to remove the necrotic cells after AMI and to repair the infarcted myocardium. To develop and evaluate innovative therapeutic approaches, we designed an *ex vivo* model that provides strong IR injuries as assessed by a 56 %-infarct size (expressed as a percentage of the left ventricel) and the release of proinflammatory cytokines at significant levels in the coronary effluents as already reported *in vivo.*

This inflammatory response is a potential therapeutic target for improving clinical post-MI since it plays a crucial role in determining infarct size and subsequent remodeling of the left ventricle. In order to protect the myocardium, numerous therapeutic strategies targeting indirectly or directly the pro-inflammatory response after AMI have been tested.

Ischemic postconditioning (PostC), considered as the gold standard in cardioprotection against IR injury, has been reported to activate a myriad of intracellular cascades resulting in the inhibition of regulated cell death and also an antiinflammatory response in order to induce a strong cardioprotective effect at one hour reperfusion (Roubille, F. et al. Delayed postconditioning in the mouse heart in vivo. Circulation 124, 1330-1336, doi:CIRCULATIONAHA.111.031864 [pii] 10.1161/CIRCULATIONAHA.111.031864 (2011)). In the *ex vivo* model of IR injury used in the present study, the PostC cardioprotective strategy decreased infarct size by 75.1 % (PostC *vs* IR, p*) and TNFα, IL-6 and CXCL1 levels by 71.7, 71.8 and 91.4% compared to IR, respectively in the coronary effluents of the isolated hearts. Altogether, these results suggest that the regulation of the inflammatory response is associated with the cardioprotection against IR injury mediated by the ischemic PostC.

In this context, strategies aiming at targeting inflammatory cytokines and chemokines in animal models have been investigated with promising results regarding the control of inflammation (Ong, S. B. et al. Inflammation following acute myocardial infarction: Multiple players, dynamic roles, and novel therapeutic opportunities. Pharmacol Ther 186, 73-87, doi:10.1016/j.pharmthera.2018.01.001 (2018)). However, most of the therapeutic trials have not shown any benefit on infarct size reduction or clinical outcomes when evaluated in AMI patients (see for review Ong, S. B. et al. Inflammation following acute myocardial infarction: Multiple players, dynamic roles, and novel therapeutic opportunities. Pharmacol Ther 186, 73-87, doi:10.1016/j.pharmthera.2018.01.001 (2018)). A significant increase in deaths in NSTEMI patients has been reported using a specific inhibitor of interleukin-1 as an adjunct of reperfusion (Morton, A. C. et al. The effect of interleukin-1 receptor antagonist therapy on markers of inflammation in non-ST elevation acute coronary syndromes: the MRC-ILA Heart Study. Eur Heart J 36, 377-384, doi:10.1093/eurheartj/ehu272 (2015)). In addition, corticosteroid or immunosuppressants have also shown disappointing results in clinical trials (Ong, S. B. et al. Inflammation following acute myocardial infarction: Multiple players, dynamic roles, and novel therapeutic opportunities. Pharmacol Ther 186, 73-87, doi:10.1016/j.pharmthera.2018.01.001 (2018)), questioning this therapeutic approach after the acute MI and suggesting that inflammation is the tip of the iceberg.

Therefore, more global therapies including MSC-based therapies have emerged as promising approaches to treat multifaceted ischemic heart disease and to restore cardiac function. Indeed, considering the pleiotropic effects of MSC that include their immunoregulatory, antifibrotic and antiapoptotic capabilities, MSC-based therapy could counteract the three main pathogenic axes of AMI and thus have been considered as a breakthrough in this incurable disorder with unmet medical need. Safety and efficacy were evaluated by the assessment of adverse events and the improvement of the left ventricular ejection fraction (LVEF) and mortality rate, respectively. Although the results obtained were marked by an important heterogeneity, MSC injection appeared to be not associated with acute adverse events and to induce an improvement of the LVEF in patients. No significant difference in mortality was reported. Other outcomes of interest were rarely studied, limiting the conclusions. In our study, MSC exerted a cardioprotective effect that was similar to that provided by PostC, considered as positive control in our study. Indeed, infarct size was decreased by 69.3% using MSC-cell therapy (PostC *vs* MSC, p=ns). In addition, MSC (6.10⁵ cells/heart) was providing a strong anti-inflammatory effect as assessed by MSD quantification of TNFα, IL-6 and CXCL1 that were decreased by 35, 75.1 and 84.6 %, respectively. Our results are in line with data reported in mouse model of AMI showing that the injection of MSC, 2 days after MI induction reduced the frequency of macrophage/monocyte including pro-inflammatory macrophages and increased the percentage of anti-inflammatory and reparative macrophages (F4/80⁺CD206⁺) both in the circulation and infarcted heart (Ben-Mordechai, T. et al. Macrophage subpopulations are essential for infarct repair with and without stem cell therapy. J Am Coll Cardiol 62, 1890-1901, doi:10.1016/j.jacc.2013.07.057 (2013); Dayan, V. et al. Mesenchymal stromal cells mediate a switch to alternatively activated monocytes/macrophages after acute myocardial infarction. Basic Res Cardiol 106, 1299-1310, doi:10.1007/s00395-011-0221-9 (2011)). Thus, MSC-based therapy counteracts one of the three main pathogenic axes of AMI, i.e. inflammation, and enhancing this immunoregulatory properties could lead to an improved therapeutic effect of MSC in AMI. Indeed, in a rat model of MI, IL-33 overexpression in MSC improves LVEF as compared to control MSC. This enhanced efficacy of MSC overexpressing IL-33 was associated with their higher capacity to promote macrophage polarization toward an anti-inflammatory phenotype *in vitro* and to reduce heart inflammation *in vivo* (Chen, Y. et al. The enhanced effect and underlying mechanisms of mesenchymal stem cells with IL-33 overexpression on myocardial infarction. Stem Cell Res Ther 10, 295, doi:10.1186/s13287-019-1392-9 (2019)). In this context, we tested, in the present study, the therapeutic role of MSC knocked down for PPARβ/δ previously described by our laboratory to exhibit enhanced capacity to inhibit both the proliferation of T lymphocytes, *in vitro,* and arthritic development and progression in CIA *in vivo* compared with naïve MSC (Luz-Crawford, P. et al. PPARbeta/delta directs the therapeutic potential of mesenchymal stem cells in arthritis. Annals of the rheumatic diseases, doi:10.1136/annrheumdis-2015-208696 (2016)). When primed with TNFα and IFNγ, MSC deficient for PPARβ/δ expressed an increased level of mediators of MSC immunosuppression including VCAM-1, ICAM-1 and nitric oxide (NO) as compared to their wild type counterpart MSC (Luz-Crawford, P. et al. PPARbeta/delta directs the therapeutic potential of mesenchymal stem cells in arthritis. Annals of the rheumatic diseases, doi:10.1136/annrheumdis-2015-208696 (2016)).

In the present study, we observed that PPARβ/δ knockdown in MSC did not modify their anti-inflammatory properties when injected in the infarcted myocardium. Indeed, both wild type and PPARβ/δ KO MSC significantly decreased the concentrations of pro-inflammatory cytokines within coronary effluents after 60 min of reperfusion after an IR protocol. Therefore, the loss of MSC therapeutic effect in myocardial ischemia-reperfusion injury reported here for PPARβ/δ KO MSC might be attributed to an impairment of others functions of MSC pivotal for their beneficial effect in AMI or reduce their survival *in vivo* by promoting their apoptosis. It is attempting to anticipate that PPARβ/δ deficiency in MSC might affect their survival rate and reduce their engraftment once injected *in vivo* since PPARβ/δ has been described to promote the survival of several cell types including cancer cells and cardiomyocytes (Palomer, X., Barroso, E., Zarei, M., Botteri, G. & Vazquez-Carrera, M. PPARbeta/delta and lipid metabolism in the heart. Biochim Biophys Acta 1861, 1569-1578, doi:10.1016/j.bbalip.2016.01.019 (2016); Li, Y. J. et al. PPAR-delta promotes survival of chronic lymphocytic leukemia cells in energetically unfavorable conditions. Leukemia 31, 1905-1914, doi:10.1038/leu.2016.395 (2017); Wang, X. et al. PPAR-delta promotes survival of breast cancer cells in harsh metabolic conditions. Oncogenesis 5, e232, doi:10.1038/oncsis.2016.41 (2016)). Indeed, the role of PPARβ/δ in vascular cells protects them from apoptosis while PPARβ/δ activation is proapoptotic when cells are deficient for cytoplasmic prostacyclin receptors (Hatae, T., et al. (2001). "Prostacyclin-dependent apoptosis mediated by PPAR delta." J Biol Chem 276(49): 46260-46267). In endothelial cells, anti-apoptotic properties of PPARβ/δ were reported and underlying mechanisms were related to of endothelial 14-3-3 activation (Liou, J. Y., et al. (2006). "Protection of endothelial survival by peroxisome proliferator-activated receptor-delta mediated 14-3-3 upregulation." Arterioscler Thromb Vasc Biol 26(7): 1481-1487; Brunelli, L., et al. (2007). "Peroxisome proliferator-activated receptor-delta upregulates 14-3-3 epsilon in human endothelial cells via CCAAT/enhancer binding protein-beta." Circ Res 100(5): e59-71). Also in cardiomyoblast cell line H9c2, activation of PPARβ/δ receptors by the selective agonist, GW501516, was described to protect the from H₂O₂-induced cell death (Pesant, M., et al. (2006). "Peroxisome proliferator-activated receptor delta (PPARdelta) activation protects H9c2 cardiomyoblasts from oxidative stress-induced apoptosis." Cardiovasc Res 69(2): 440-449). PPARβ/δ are highly expressed by MSC (Luz-Crawford, P., et al. (2016). "PPARbeta/delta directs the therapeutic potential of mesenchymal stem cells in arthritis." Ann Rheum Dis 75(12): 2166-2174) but their C anti-apoptotic and cardioprotective properties has never been investigated.

In conclusion, our study shows that the cardioprotective effects of MSC are lost when PPARβ/δ receptor is lacking, despite a sustained anti-inflammatory effect. This suggests that the enhancement of MSC immunoregulatory properties to improve their beneficial effect in AMI should not be the only or main approach to overcome phase III clinical trial inconsistencies (Jeong, H. et al. Mesenchymal Stem Cell Therapy for Ischemic Heart Disease: Systematic Review and Meta-analysis. International journal of stem cells, doi:10.15283/ijsc17061 (2018); Roncalli, J. et al. Intracoronary autologous mononucleated bone marrow cell infusion for acute myocardial infarction: results of the randomized multicenter BONAMI trial. Eur Heart J 32, 1748-1757, doi:10.1093/eurheartj/ehq455 (2011)), but rather it should be considered concomitantly with the enhancement of their survival once administrated to the patient.

### Example 2: Peroxisome Proliferator-Activated Receptor Beta/Delta agonist enhances the anti-apoptotic and therapeutic properties of mesenchymal stem cells in myocardial ischemia-reperfusion injury

Following the results obtained in Example 1 using KO MSC cells, the effect of pretreatment of MSCs with a PPARβ/δ agonist on their anti-apoptotic and therapeutic properties in myocardial ischemia-reperfusion injury was tested.

### Materials and Methods

### Animal experiments

All experiments were carried out on C57BL/6J mice *(Charles River laboratories*) in accordance with the European Communities Council directive of November 1986 and conformed to the "Guide for the Care and Use of Laboratory Animals" published by the US National Institutes of Health (NIH publication 8th Edition, 2011).

### Langendorff ex vivo studies

C57Bl6 male mice were anesthetized with a first intramuscular injection of an anesthetic cocktail comprising ketamine (14 mg/kg, Imalgène^{®}; *Merial)* and xylazine (14 mg/kg, Rompun^{®}; *Bayer),* and by a second injection of pentobarbital (76.6 mg/kg; *Sanofi-Aventis).* After sternotomy, the heart was excised, quickly cannulated through the aorta and mounted on a homemade Langendorff system. It was retrogradely perfused at pressure- and temperature-constant (37°C) Tyrode solutions.

### Ischemia-reperfusion protocols

On the Langendorff system, the heart was subjected to ischemia-reperfusion (IR) protocol comprising, after a 15 min-stabilization period of Tyrode perfusion, a 30 min-period of no-flow to induce global ischemia). Reperfusion was achieved by restoring the perfusion during 60 min with the Tyrode solution alone (IR control) or with MSC cell in Tyrode solution (MSC) administrated during reperfusion. Ischemic postconditioning was induced at the end of global ischemia by the application of a conditioning stimulus comprising 3 cycles of 1 min-reperfusion / 1 min-ischemia, followed by 54 min of reperfusion. For the *ex vivo* evaluation of MSC-based cell therapy, 80 to 150 mL of MSC (mean value 120 mL) cells prepared in the Tyrode solution at various concentrations (2500, 5000 et 20000 cells/mL) were perfused retrogradely via the aorta and the coronary vessels in isolated hearts during the 60 min-reperfusion phase.

### Infarct size assessment

LV were sliced transversally into 1-mm-thick sections and incubated in a 1% solution of 2,3,5-triphenyltetrazolium chloride (TTC, *Sigma-Aldrich)* for 15 min at 37°C. After fixation in a 4% paraformaldehyde-PBS solution, the slices were weighted and each side was photographed (*Olympus* camera). Infarcted area was measured by planimetry with ImageJ software *(U. S. National Institutes of Health)* and expressed as a percentage of the left ventricle.

### MSC culture

MSC isolation and amplification of murine MSCs was carried out in conditions previously described (Bouffi, C., Bony, C., Courties, G., Jorgensen, C. & Noel, D. IL-6-dependent PGE2 secretion by mesenchymal stem cells inhibits local inflammation in experimental arthritis. PLoS One 5, e14247 (2010)). MSCs were then seeded at a density of 0.5×10⁶cells per cm² in minimum essential medium (MEM)-α supplemented with 10% fetal bovine serum (FBS), 2 mM glutamine, 100 U/mL penicillin, 100 mg/mL streptomycin and 2 ng/ml human basic fibroblast growth factor (bFGF). The phenotypic and differentiation potential of MSCs was performed as previously (Bouffi, C., Bony, C., Courties, G., Jorgensen, C. & Noel, D. IL-6-dependent PGE2 secretion by mesenchymal stem cells inhibits local inflammation in experimental arthritis. PLoS One 5, e14247 (2010)). Briefly, for the phenotypic characterization, MSCs were incubated during 20 minutes with conjugated monoclonal antibodies from BD Biosciences (Le Pont de Claix, France) in PBS supplemented with 0.1% bovine serum albumin. Regarding the differentiation of MSCs into adipocytes, osteoblasts and chondrocytes, we relied on the inductive conditions already reported (Bouffi, C., Bony, C., Courties, G., Jorgensen, C. & Noel, D. IL-6-dependent PGE2 secretion by mesenchymal stem cells inhibits local inflammation in experimental arthritis. PLoS One 5, e14247 (2010)). Adipogenic differentiation of MSC was assessed by the analysis of lipid droplets formation after Oil red O staining and by RT-qPCR. MSC chondrogenic differentiation was evaluated by RT-qPCR and the osteogenic differentiation was measured by RT-qPCR and the mineralization of the extracellular matrix.

### MSC pretreatments with PPARβ/δ agonist and antagonists and labeling with CM-DiI.

MSCs were pre-incubated for 24 hours with either PPARβ/δ antagonists GSK3787 (0.1 and 1 µM), PPARβ/δ inverse agonist GSK0660 (0.1 and 1 µM) or PPARβ/δ agonist GW0742 (0.1, 1 and 5 µM) before to be washed with PBS and used in co-cultures experiments or injected in the myocardium. When indicated, MSCs were labeled with the fluorescent cell-tracer CM-DiI (Molecular Probes). MSC were collected and suspended in 5 mL PBS containing CM-Dil (10⁷ cells/10 µg) prior to be incubated at 37°C for 5 minutes followed by 15 minutes at 4°C, in the dark. Labeled MSCs were washed 2 times in PBS, resuspended in PBS and maintained at 4°C prior to be injected in the myocardium.

### Cardiomyocyte and MSC co-cultures

H9c2, rattus norvegicus cardiomyocytes, were cultured in Dulbecco's modified Eagle's medium (DMEM), supplemented with 10% fetal bovine serum (FBS), 100 Units/mL penicillin, and 100 µg/mL streptomycin. To induce apoptosis, H9c2 were cultured in a minimal medium (without FBS) containing 350 µM of H₂O₂ during 4 hours. In order to evaluate the effect of MSCs on cardiomyocytes, a co-culture assay was designed using the transwell system to avoid cell-cell contact. 15,000 cardiomyocytes (lower part) were plated per well of a 12-well dish with or 15,000 MSC (upper part) in presence of 350 µM of H₂O₂. Two different protocols of co-culture were performed. In protocol 1, H9c2 and co-cultured MSC were exposed to H₂O₂ (with serum deprivation). In protocol 2, H9c2 were exposed to H₂O₂ in minimal medium during 4 hours and after a wash, cells were co-cultured in complete medium with MSC. In protocol 3, H9c2 were cultured in presence of H₂O₂ during 4 hours in regular medium deprived in serum, which was continued for an extra 1 hour. Then the co-culture of H9c2 with MSC was performed in complete medium for 1 hour. At the end of all protocols, H9c2 were recovered for apoptosis and RT-qPCR analysis.

### DNA fragmentation assay

DNA fragmentation was quantified *in vitro* on cell lysates with an enzyme-linked immunosorbent assay kit (Cell Death ELISA; *Roche Diagnostics)* designed to measure of histone-complexed DNA fragments (mono- and oligonucleosomes) out of the cytoplasm of cells after the induction of apoptosis.

### RT-qPCR

Isolation of total RNA for each sample was performed using the RNeasy Mini Kit (Qiagen, Courtaboeuf). The quantity and quality of the RNA extracted were defined by using a NanoDrop ND-1000 spectrophotometer (NanoDrop ND, Thermo Fisher Scientific). Then, using the SensiFAST cDNA synthesis kit (Bioline), cDNA was synthesized by reverse transcribing 500 ng RNA into cDNA. Using the SensiFAST^{™} SYBR (Bioline) and a LightCycler^{®} 480 Detection system Quantitative following manufacturer's recommendations, PCR were performed. Primers specific for *angpl4, pdk4, bcl2, hgf, pdgf, nrf2* were designed using the Primer3 software. Data were normalized to the housekeeping gene ribosomal protein S9 (RPS9) and the values were expressed as relative mRNA level of specific gene expression as calculated using the 2^{-ΔCt} method.

### Immunoblotting

Tissue samples (left ventricle) were rapidly frozen in liquid nitrogen after the end of *ex vivo* IR protocols. Samples were homogenized with a grinder in RIPA buffer [50 mM Tris (*Sigma-Aldrich*), 150 mM NaCl *(Sigma-Aldrich),* 1% Triton X-100 *(Sigma-Aldrich),* 0.1% sodium dodecyl sulfate (*Sigma-Aldrich*) pH 8.0] supplemented with EDTA-free protease and phosphatase inhibitor (Halt^{™} Protease and phosphatase single-use inhibitor Cocktail-100X, *Thermoscientific).* After centrifugation, pellets were resuspended in RIPA buffer for protein purification and incubated for 1 hour at 4°C. Protein concentrations were determined with the bicinchoninic acid (BCA) protein assay kit *(Pierce).* Samples (25 µg) of protein were resolved by SDS polyacrylamide gel electrophoresis (4-20% mini-Protean^{®}TGX^{™} precast gels; *Biorad)* and transferred to nitrocellulose (Trans-Blot^{®}Turbo^{™}; *Biorad).* The following antibodies and suppliers were used: 1) *EMD Millipore:* anti-caspase 3 (Upstate 06-735), 2) *Cell Signaling Technology:* anti-phospho ERK1,2; anti-ERK1,2; anti-phospho AKT; anti-AKT; β-Tubulin; anti-α-actinin; vinculin (E1E9V), GAPDH, 3) *Jackson ImmunoResearch:* horseradish peroxidase-conjugated anti-rabbit. Protein bands were visualized by enhanced chemiluminescence method using an ECL kit (SuperSignal^{™} West Pico chemiluminescent Substrate; *Thermo Scientific^{™}*). Densitometry analysis was performed using Chemidoc^{™} *(Biorad)* and ImageJ software (*U. S. National Institutes of Health).* Signal intensities of each protein band were corrected with the corresponding value obtained for the loading controls (tubulin, vinculin, GAPDH or α-actinin) and then normalized with the mean value obtained for the IR control conditions.

### Immunochemistry

At the end of ex vivo experiments, left ventricles (LV) were fixated in formol and embedded in paraffin. Each left ventricle was cut from the apex through the base (4 µm sections each 150 µm). Paraffin-embedded sections were deparaffinised then rehydrated through an alcohol gradient. Sections were mounted in Mowiol (Biovalley). LV Sections were scanned by the Zeiss Axio Scan Z1 slide scanner (Zeiss, Oberkochen, Germany). Scanning was done with the x20 objective. Images were analyzed using ImageJ.

### Statistical analysis

Statistical analysis was performed only for n ≥ 5 independent experiments. Data (mean ± SD) were analyzed with ANOVA parametric test after confirmation of the normality of the population (Anderson-Darling Test for normal distribution). In case of non-conformity, nonparametric Kruskal-Wallis followed by Dunn's post hoc test for multiple comparison or Mann-Whitney for two groups were used. Data were analyzed with GraphPad Prism (version 8.3.0 for MacOs, GraphPad Software, San Diego, California USA, www.graphpad.com). Statistical significance was noted as ns for p> 0.05,* for p<0.05, ** for p<0.01, *** for p<0.001 and **** for p<0.0001.

### Results

### The cardioprotective effect of MSC administered during reperfusion in an ex vivo model of global ischemia depends on PPARβ/δ

First, we phenotypically and functionally characterized MSC used for the study as previously described (Bouffi, C., Bony, C., Courties, G., Jorgensen, C. & Noel, D. IL-6-dependent PGE2 secretion by mesenchymal stem cells inhibits local inflammation in experimental arthritis. PLoS One 5, e14247 (2010)). We showed that MSCs were negative for CD11b and CD45 and were positive for markers expressed on MSCs such as CD44 and Sca-1 (data not shown). After induction of MSC differentiation towards the three specific lineages, MSC gave rise to: 1) chondrocytes as shown by the expression of collagen type II (Col2); 2) adipocytes characterized by the expression of *pparγ* and *lpl* and the formation of lipid droplets, and 3) osteoblasts characterized by the expression of osteocalcin and alkaline phosphatase and Alizarin Red S staining (data not shown). In order to evaluate the protective effect of MSC in our *ex vivo* model of IR injury, MSC were administered at various concentrations (2500, 5000 et 10000 cells/mL in a Tyrode solution) during the reperfusion phase in isolated hearts subjected to global ischemia (see protocol, **Figure 5A). Figure 5B** presents the U-curve obtained for the dose-response of the cardioprotective effect of MSC. The 5,000 cells/mL concentration was identified as the dose providing the optimal efficacy similar to that provided by the PostC stimulus, which was considered as the positive control in our experiment (28.13 ± 4.69, n=7 for PostC vs 30.27 ± 5.89, for MSC 5,000; n=12; p $>0.999). In addition, neither the 2,500 cells/mL dose (38.64 ± 5.36, n=11 vs 46.51 ± 6.28, n=11 for IR; pns= 0.80) nor the largest one tested, 10,000 cells/mL (50.28 ± 11.09, n=6 for MSC 10,000 vs 46.51 ± 6.28, n=11 for IR; pns>0.999) produced a significant decrease in infarct size compared to IR.

Since, we demonstrated in previous studies the pivotal role of PPARβ/δ expression on MSC functions including their differential potential and immunoregulatory properties we addressed here its role on MSC cardioprotective effect (Scholtysek, C. et al. PPARβ/δ governs Wnt signaling and bone turnover. Nat Med 19, 608-613, doi:10.1038/nm.3146 (2013; Luz-Crawford, P. et al. PPARbeta/delta directs the therapeutic potential of mesenchymal stem cells in arthritis. Ann Rheum Dis 75, 2166-2174, doi:10.1136/annrheumdis-2015-208696 (2016);Contreras-Lopez, R. A. et al. PPARβ/δ-dependent MSC metabolism determines their immunoregulatory properties. Sci Rep 10, 11423, doi:10.1038/s41598-020-68347-x (2020)). To that end, MSC were pretreated with either PPARβ/δ antagonist or agonist pharmacological agents prior to their administration in the ex vivo model of IR injury (5,000 cells/mL). **Figure 5C** shows that the cardioprotection was decreased when MSC (5,000 cells/mL) were pretreated during 24 hours with GSK0660 (1 µM) prior to their administration in the ex vivo ischemic heart during the reperfusion phase (37.78 ± 5.31, n=9 for MSC+GSK0660 vs 30.27 ± 5.89, n=12 for MSC; p#=0.019). According to this result, we investigated whether the use of GW0742 PPARβ/δ agonist at various doses (0.1, 1 and 5 µM) was associated with an improvement of the cardioprotection afforded by MSC administered at the lower dose (2,500 cells/ml) that was not protective by itself **(****Figure 5D****).** First, we validated the effect of GW0742 PPARβ/δ agonist pretreatment on cultured MSC and found that the agonistic effect of GW0742 was associated with the increased expression levels of angpl4 and pdk4 target genes allowing to demonstrate the agonist functionality (data not shown). Then, we showed that the MSC pretreatment with various concentrations of PPARβ/δ agonist induced their cardioprotective effect at the concentration of 1 µM (25.77 ± 4.68, n=7 for MSC + GW0742 1 µM vs 46.51 ± 6.28, n=11 for IR; p****<0.0001) while no beneficial effect was observed at 0.1 and 5 µM. Pre-treating the cells with GW0472 (1 µM) before administration at 2500 c/mL allowed to induce the same cardioprotective effect as the one observed using 5000 c/mL naïve MSC **(****Figure 5E****).** This improvement of the therapeutic effect of PPARβ/δ-primed MSC was associated with an increased number of MSC grafted in the infarcted myocardial tissue as assessed by immunohistology experiments using DiI-labeled MSC showing that the percentage of fluorescence was increased by 3.5 times upon GW0472 pre-treatment **(****Figure 5F****).**

### Increased resistance of PPARβ/δ-primed MSC to simulated IR

Since histologic experiments revealed an increased number of MSC in the infarcted area of myocardium when MSC were primed with PPARβ/δ agonist, we investigated first the resistance of primed-MSC to the IR stress. To do this, MSC were submitted to an IR-simulated stress *in vitro* using various concentrations of H₂O₂ (100, 250, 350, 500 and 750 µM; see the protocol on **Figure 6A****)** for 4 hours. Apoptosis quantification was assessed by the mean of specific DNA fragmentation measurement for the different groups of treatment. We found that specific DNA **fragmentation** was induced by H₂O₂ in MSC exposed to 100 µM of H₂O₂ with a score that plateaued from 100 to 750 µM **(****Figure 6B****).** The concentration of 250 µM of H₂O₂ was chosen because it was the lowest dose providing the maximal effect compared to the untreated MSC (p****). To determine the role of PPARβ/δ on the resistance of MSC to H₂O₂, we pretreated them with different concentrations (0.1 and 1 µM) of PPARβ/δ antagonist and agonist for 24 hours before the H₂O₂-stress induction (250 µM for 4 hours). MSC pretreatment **with** 0.1 µM of PPARβ/δ **agonist GW0742 did not modify the capacity of MSC to resist against** oxidative **stress (****Figure 6C****). However,** the pretreatment with 1 µM of PPARβ/δ **agonist GW0742 enhanced significantly** MSC resistance compared to naive MSC exposed to H₂O₂ **(p*).** The pretreatment with 1 µM of PPARβ/δ **antagonist GSK0660 impaired MSC resistance** against H₂O₂-induced oxidative without reaching significance **(****Figure 6C****).** This enhanced resistance to apoptosis induced by PPARβ/δ **agonist pretreatment on MSC was confirmed by a significant increase in bcl2 gene expression level in particular after pre-treatment with GW0742 at 0.1 µM (p**). Conversely, the pretreatment of MSC with 1 µM of GSK0660 significantly reduced bcl2 gene expression level compared to control** H₂O₂ treated MSC (p*; **Figure 6D****).** Altogether, these results revealed that the activation of PPARβ/δ protects MSC from apoptosis provided by H₂O₂ -induced simulated IR stress.

### PPARβ/δ-priming increases the protective effects of MSC on cardiomyocytes exposed to an IR-like oxidative stress

To determine the role of PPARβ/δ on MSC-induced protective effect on cardiomyocytes we designed and developed three protocols in order to mimic the stress received by MSC when transplanted into infarcted tissues. The first protocol consisted in co-culturing naive or primed MSC with H9c2 exposed to 350 µM of H₂O₂ for 4 hours using transwell. The second protocol consisted in exposing H9c2 to 350 µM of H₂O₂ for 4 hours before to co-culture them with naive or primed MSC in a complete medium. The third protocol consisted in exposing H9c2 to 350 µM of H₂O₂ for 4 hours followed by serum deprivation for 1 hour before to co-culture them with naive or primed MSC in a complete medium **(****Figure 7A****).** With the first protocol, we found that MSC pretreated with 1 µM of GSK0660 or GSK3787 lose their capacity to protect cardiomyocytes from apoptosis as compared to naive MSC. By contrast, MSC pretreated with 0.1 and 1 µM of PPARβ/δ **agonist GW0742 exhibited a higher anti-apoptotic effect on** cardiomyocytes exposed to an H₂O₂-induced oxidative stress as compared to naive MSC **(****Figure 7B****).** With the second protocol, we showed that MSC pretreatment with 1 µM of GSK0660 or GSK3787 did not change MSC protective properties on H₂O₂-stressed cardiomyocytes. However, MSC pretreatment with 1 µM of PPARβ/δ **agonist GW0742 induced a significant protective effect (MSC, n=18 vs MSC/1 µM GSK07342, n=10; p***=0.0008) on** cardiomyocytes exposed to an IR-like oxidative stress as compared to naive MSC **(****Figure 7C****).** For the third protocol, MSC pretreated with 1 µM of GSK0660 or GSK3787 lose their capacity to protect cardiomyocytes from apoptosis as compared to naive MSC. PPARβ/δ **agonist GW0742-pretreatment (1 µM) induced a significant protective effect (MSC, n=11 vs MSC/1 µM GSK07342, n=6; p*=0.004326) on** cardiomyocytes exposed to an IR-like oxidative stress as compared to naive MSC **(****Figure 7D****).** Altogether, these results revealed that MSC pretreatment with PPARβ/δ agonist prior to their co-culture with injured cardiomyocytes enhances their anti-apoptotic properties on cardiomyocytes *in vitro.*

### PPARβ/δ-priming inverses the repressive effects of the IR-like oxidative stress on MSC paracrine functions

MSC transplantation inhibits IR-induced apoptosis through their capacity to release anti-apoptotic factors including VEGF (Scott, R. C. et al. Targeting VEGF-encapsulated immunoliposomes to MI heart improves vascularity and cardiac function. Faseb j 23, 3361 - 3367, doi:10.1096/fj.08-127373 (2009); Ruvinov, E., Leor, J. & Cohen, S. The promotion of myocardial repair by the sequential delivery of IGF-1 and HGF from an injectable alginate biomaterial in a model of acute myocardial infarction. Biomaterials 32, 565-578, doi:10.1016/j.biomaterials.2010.08.097 (2011)) and PDGF (Xu, B., Luo, Y., Liu, Y., Li, B. Y. & Wang, Y. Platelet-derived growth factor-BB enhances MSC-mediated cardioprotection via suppression of miR-320 expression. Am J Physiol Heart Circ Physiol 308, H980-989, doi:10.1152/ajpheart.00737.2014 (2015)) also described for their cardiac regenerative capability when applied to AMI model. We thus tested the effect of the H₂O₂-induced oxidative stress on the capacity of MSC to regulate the transcript levels of these factors **(****Figure 8A****)** and found that H₂O₂-stressed MSC exhibited an enhanced ability to up-regulate *vegf-a* transcripts and to down-regulate those of *hgf* and *pdgf* **(****Figure 8B-D****).** We evidenced in MSC pretreated with 0.1 or 1 µM of GSK0660 that amounts of *vegf-a* transcripts were significantly decreased (p*** and p*, respectively; **Figure 8B****).** A similar tendency was observed for *hgf* and *pdgf* **(****Figure 8C, D****).** By contrast, while MSC pretreatment with 0.1 and 1 µM of PPARβ/δ **agonist GW0742 did not modify vegf-a and hgf transcription levels of MSC (****Figure 8B, C****), it significantly increased the expression of pdgf when applied at the concentration of 1** µM (p*; **Figure 8D****).**

### PPARβ/δ-priming increases the cardioprotective effects of MSC ex vivo

The potent anti-apoptotic effect of the PPARβ/δ-primed MSC on cardiomyocytes was further confirmed on whole hearts. Western blot analysis in hearts subjected *ex vivo* to IR injury allowed to show that MSC-treatment induced a decreased activation of caspase 3 compared to the non-treated IR condition **(****Figure 9A****).** When MSC were pretreated during 24 hours with the **GW0742** PPARβ/δ agonist (1 µM), this inhibition of caspase 3 cleavage was further increased **(****Figure 9B****).** By contrast, pre-treating MSC with GSK0660 PPARβ/δ antagonist prior to their injection in the *ex vivo* IR heart at reperfusion, restored full activation of caspase 3 at the same level than in IR control conditions.

In addition, evaluation of the phosphorylation patterns of ERK1/2 **(****Figure 9C****)** and AKT **(****Figure 9D****)** pro-survival kinases showed a significantly increased phosphorylation ratio pAkt/Akt in MSC-treated *versus* non-treated IR hearts when MSC were pre-treated with **GW0742** PPARβ/δ agonist (1 µM) prior to their administration during reperfusion. By contrast, there was no change in the pERK1/2 /ERK1/2 ratio when naive MSC were administered in the post-ischemic phase of the IR protocol.

### Discussion and conclusions

Our study is the first to demonstrate that PPARβ/δ activation protect MSC from apoptosis, enhances their anti-apoptotic and therapeutic properties against myocardial ischemia-reperfusion injury. For this study, we have developed an *ex vivo* model of global ischemia followed by reperfusion (IR protocol) to evaluate and compare the therapeutic effects of naive *versus* PPARβ/δ-primed MSC. MSC administered during the reperfusion phase in hearts subjected to the IR protocol allowed to decrease infarct size. When MSC were pre-treated with PPARβ/δ antagonist during 24 hours prior to their administration, their cardioprotective effect was significantly decreased. Pre-treatment with PPARβ/δ agonist increased significantly MSC-induced cardioprotection, which was associated with a significant increased number of cells detected in the ventricular wall. We evidenced *in vitro* that PPARβ/δ pre-activation allowed to increase MSC survival and resistance against oxidative stress. This increased resistance of MSC to H₂O₂-induced stress was associated to a decreased apoptosis rate when MSC were primed with PPARβ/δ agonists and also by an increased amount of *bcl2* transcripts. In addition, PPARβ/δ-primed MSC had a more potent anti-apoptotic therapeutic effect on H₂O₂-stressed cultured cardiomyocytes than naïve MSC. In the whole heart, our results revealed also that the anti-apoptotic effects of PPARβ/δ-primed MSC was more efficient than that provided by naive MSC as assessed by western blot analysis of caspase 3 activation. Accordingly, the phosphorylation ratio of AKT pro-survival kinase was also increased in IR hearts treated by PPARβ/δ-primed *versus* naive MSC.

Reperfusion, even if considered as a double-edged sword, is the only treatment recommended for patients with acute myocardial infarction (Braunwald, E. & Kloner, R. A. Myocardial reperfusion: a double-edged sword? J Clin Invest 76, 1713-1719, doi:10.1172/JC1112160 (1985)). Despite obvious beneficial effects in resolving the ischemic insult, revascularization of the culprit artery leads to an abrupt return of oxygen in the threatened myocardial tissue. Lethal reperfusion injury corresponds to the death of cardiac cells viable at the end of the ischemic period occurring at the onset of reperfusion and that culminates in apoptotic death (Piper, H. M. & Garcia-Dorado, D. Prime causes of rapid cardiomyocyte death during reperfusion. Ann Thorac Surg 68, 1913-1919, doi:10.1016/s0003-4975(99)01025-5 (1999); Zhao, Z. Q. et al. Progressively developed myocardial apoptotic cell death during late phase of reperfusion. Apoptosis 6, 279-290 (2001)). Many regulated forms of cell death, apoptosis, necrosis, necroptosis and autophagy, can be activated in cardiac cells during ischemia-reperfusion injury. Apoptosis and necrosis are induced mainly in case of severe insult and autophagy contributes to ensure cell survival when lesions are more moderate (Galluzzi, L. et al. Molecular mechanisms of cell death: recommendations of the Nomenclature Committee on Cell Death 2018. Cell death and differentiation 25, 486-541, doi:10.1038/s41418-017-0012-4 (2018)). Targeting regulated cell death pathways after an ischemic event appeared as a main strategy to prevent reperfusion injury and to limit infarct size (Boisguerin, P. et al. A novel therapeutic peptide targeting myocardial reperfusion injury. Cardiovasc Res 116, 633-644, doi:10.1093/cvr/cvz145 (2020)). Anti-apoptotic strategies allows to inhibit specifically reperfusion-induced apoptotic cell death, providing long-term cardioprotective effects as evidenced recently by our group in the mouse heart (Covinhes, A. et al. Anti-apoptotic peptide for long term cardioprotection in a mouse model of myocardial ischemia-reperfusion injury. Sci Rep 10, 18116, doi:10.1038/s41598-020-75154-x (2020)). The *ex vivo* model used in our study was designed to produce a 46.5 % infarct size (expressed as a % of the LV) inducing a strong pro-apoptotic stress as evidenced by *western blot* analysis of cleaved caspase 3 expression (SHAM, n=6 *vs* IR, n=10; p**=0.0047; data not shown). MSC administered at reperfusion in this *ex vivo* model of isolated perfused heart provided a 35 %-decrease in infarct size. We observed a U-shaped curve of cardioprotection already evidenced with *in vivo* data, clearly showing that MSC-induced cardioprotection is dose-dependent as reported in clinical trials. Indeed, in AMI patients, a recent study showed that only doses less than 10⁷ MSC administered within 1 week enhanced the systolic function of left ventricle whereas administration of more than 10⁷ cells after 1 week had the opposite effect (Wang, Z. et al. Rational transplant timing and dose of mesenchymal stromal cells in patients with acute myocardial infarction: a meta-analysis of randomized controlled trials. Stem Cell Res Ther 8, 21, doi:10.1186/s13287-016-0450-9 (2017)). In agreement with this study, a U-shaped curve for cardioprotective effects of MSC in the treatment of chronic advanced ischemic heart failure was reported indicating inferior outcomes at the highest dose of cells (Bartunek, J. et al. Congestive Heart Failure Cardiopoietic Regenerative Therapy (CHART-1) trial design. Eur J Heart Fail 18, 160-168, doi:10.1002/ejhf.434 (2016)). The U-shape curve allowed to identify an optimal dose providing a maximal cardioprotective effect (reperfusion with a 5000 c/mL during 60 minutes). The corresponding amount of cells that was administered per heart was around 7.5.10⁵ cells, which corresponds to a relatively low dose. Using a higher dose of MSC induced the loss of cardioprotection. These results are in line with the observation that in humans after administration of higher doses (more than 10⁷ cells) deleterious effects appeared (Wang, Z. et al. Rational transplant timing and dose of mesenchymal stromal cells in patients with acute myocardial infarction: a meta-analysis of randomized controlled trials. Stem Cell Res Ther 8, 21, doi:10.1186/s13287-016-0450-9 (2017)). Interestingly, we observed also that MSC primed with a PPARβ/δ agonist allowed to decrease the dose of MSC injected in the heart by two. Indeed, in our study, the cardioprotective effect observed *ex vivo* using PPARβ/δ-primed MSC injected at a dose of 3.10⁵ MSC was similar to that obtained with naive MSC administered at the optimal dose (6.10⁵ MSC). MSC-induced cardioprotection evidenced *ex vivo* was associated with a decreased amount of cleaved caspase 3 measured in the left ventricle as already reported for MSC injected in the injured myocardium (Ward, M. R., Abadeh, A. & Connelly, K. A. Concise Review: Rational Use of Mesenchymal Stem Cells in the Treatment of Ischemic Heart Disease. Stem Cells Transl Med 7, 543-550, doi:10.1002/sctm.17-0210 (2018)). MSC priming with PPARβ/δ agonist exacerbated the anti-apoptotic properties evidenced in hearts *ex vivo* using naive MSC. This result was further confirmed by *in vitro* experiments showing the increased efficacy of primed MSC to inhibit H₂O₂-induced apoptosis in co-cultured cardiomyocytes. Using various protocols mimicking the influence of the environment on MSC at the time of transplantation, we evidenced that PPARβ/δ agonist-priming allowed to increase the therapeutic properties of MSC. In protocol 2 and 3, by contrast to the protocol 1, the co-culture of MSC with H9c2 was realized in presence of complete medium, assuming that *in vivo,* MSC will be delivered during the reperfusion period in presence of oxygenated blood at the site of the infarcted area. However, in the two protocols, MSC have never been exposed to H₂O₂, which does not represent clinical situations. Therefore, it would be of interest to investigate the cardioprotective effect of primed MSC in a supplemental protocol. In this protocol, H9c2 will be exposed to an oxidative stress prior to be co-cultured with MSC still facing a H₂O₂-induced stress during 4 hours before to switch to a complete medium to mimic the reentry of oxygenated blood into the ischemic heart that will host both stressed cardiomyocytes and primed MSC.

Recently, in an *in vivo* proof-of-concept study focused on murine arthritis, our group has demonstrated that PPARβ/δ expression level predicts MSC immunomodulatory potential and that its down-regulation enhances their therapeutic anti-inflammatory effects (Luz-Crawford P, Ipseiz N, Espinosa-Carrasco G, et al. PPARβ/δ directs the therapeutic potential of mesenchymal stem cells in arthritis Annals of the Rheumatic Diseases 2016;75:2166-2174). In the present study, downregulation of PPARβ/δ activity prior to their *ex vivo* administration decreased the MSC cardioprotective effects in IR injured hearts. By the same way, pretreating MSC with PPARβ/δ agonist (GW0742, 1 µM) potentiated this cardioprotective effect. Indeed, we demonstrates that stimulation of PPARβ/δ receptors in MSC administered at 2500 cells/mL (providing a weak and non-significant beneficial effects) during reperfusion in infarcted hearts allowed to unmask their cardioprotective properties. *In vitro,* we demonstrate here for the first time in MSC that PPARβ/δ preconditioning was associated with their enhanced capacity to be protected from apoptosis when they were subjected to an H₂O₂ stress. In several differentiated cell types including H9c2 cardiomyoblasts, endothelial cells, neurons or keratinocytes, PPARβ/δ stimulation has been described to provide anti-apoptotic effects in *in vitro* experiments (Liou, J. Y., et al. Protection of endothelial survival by peroxisome proliferator-activated receptor-delta mediated 14-3-3 upregulation. Arteriosclerosis, thrombosis, and vascular biology 26, 1481-1487, doi:10.1161/01.ATV.0000223875.14120.93 (2006); Pesant, M. et al. Peroxisome proliferator-activated receptor delta (PPARdelta) activation protects H9c2 cardiomyoblasts from oxidative stress-induced apoptosis. Cardiovasc Res 69, 440-449, doi:10.1016/j.cardiores.2005.10.019 (2006); Brunelli, L., et al. Peroxisome proliferator-activated receptor-delta upregulates 14-3-3 epsilon in human endothelial cells via CCAAT/enhancer binding protein-beta. Circ Res 100, e59-71, doi:10.1161/01.RES.0000260805.99076.22 (2007)).

In endothelial cells, anti-apoptotic properties of PPARβ/δ were shown to be mediated through the increased expression of endothelial 14-3-3 that binds the pro-apoptotic factor Bad, leading then to an increased protection against oxidative stress (Liou, J. Y., et al. Protection of endothelial survival by peroxisome proliferator-activated receptor-delta mediated 14-3-3 upregulation. Arteriosclerosis, thrombosis, and vascular biology 26, 1481-1487, doi:10.1161/01.ATV.0000223875.14120.93 (2006); Brunelli, L., et al. Peroxisome proliferator-activated receptor-delta upregulates 14-3-3 epsilon in human endothelial cells via CCAAT/enhancer binding protein-beta. Circ Res 100, e59-71, doi:10.1161/01.RES.0000260805.99076.22 (2007)).

Other anti-apoptotic mechanisms have also been highlighted following the activation of PPARβ/δ receptors, such as the AKT1 pathway. Indeed, Di-Pοï and collaborators have shown that the activation of PPARβ/δ in keratinocytes prevents apoptosis by increasing the transcription of *ilk (integrin-linked kinase)* and *pdk1 (3-phosphoinositide-dependent kinase-1)* (Di-Poï, N., et al. Antiapoptotic role of PPARbeta in keratinocytes via transcriptional control of the Akt1 signaling pathway. Mol Cell 10, 721-733, doi:10.1016/s1097-2765(02)00646-9 (2002)). Gamdzyk's team has shown in the context of neuronal stress that the activation of PPARβ/δ by its agonist GW0742 inhibits TXNIP *(Thioredoxin interacting protein),* responsible for the activation of the pro-apoptotic pathway ASK1/p38 MAPK (Gamdzyk, M. et al. Role of PPAR-β/δ/miR-17/TXNIP pathway in neuronal apoptosis after neonatal hypoxic-ischemic injury in rats. Neuropharmacology 140, 150-161, doi:10.1016/j.neuropharm.2018.08.003 (2018)).. In the context of MSC, the decreased rates of apoptosis observed in presence of H₂O₂-induced stress could be of major importance to explain the augmentation of their therapeutic properties. Apoptosis is a well-known mechanism of cell death affecting the viability of MSCs in particular when MSC are transplanted in injured tissues, such as damaged hearts after IR injury (Zhu, W et al. Hypoxia and serum deprivation-induced apoptosis in mesenchymal stem cells. Stem cells 24, 416-425, doi:10.1634/stemcells.2005-0121 (2006)). Several strategies were developed to inhibit apoptosis of MSC after grafting to help them to survive hypoxia-reoxygenation injury such as heat-shock treatment (Gao, F. et al. Heat shock protein 90 protects rat mesenchymal stem cells against hypoxia and serum deprivation-induced apoptosis via the PI3K/Akt and ERK1/2 pathways. J Zhejiang Univ Sci B 11, 608-617, doi:10.1631/jzus.B1001007 (2010)). or a hypoxia-regulated Heme oxygenase-1 (HO-1) vector modification (Tang, Y. L. et al. Improved graft mesenchymal stem cell survival in ischemic heart with a hypoxia-regulated heme oxygenase-1 vector. Journal of the American College of Cardiology 46, 1339-1350, doi:10.1016/j.jacc.2005.05.079 (2005)) of MSC. Among the factors impacting negatively the therapeutic effect of MSC, the poor survival after cell transplantation is of major importance. So, with the aim to improve their therapeutic efficacy against IR injury, MSC resistance to oxidative stress should be increased (Li, L., Chen, X., Wang, W. E. & Zeng, C. How to Improve the Survival of Transplanted Mesenchymal Stem Cell in Ischemic Heart? Stem Cells Int 2016, 9682757, doi:10.1155/2016/9682757 (2016)). Our study evidenced that PPARβ/δ agonist-priming allowed to increase MSC resistance to H₂O₂-induced oxidative stress *in vitro.* Furthermore, our RT-qPCR experiments showed that MSC-increased resistance to H₂O₂-induced stress upon PPARβ/δ-priming was associated with an increased amount of anti-apoptotic *bcl2* gene transcripts supporting the decreased rates of apoptosis.

Moreover, we observed an upregulation of *vegf (vascular endothelial growth factor)* gene. VEGF was reported to activate the proangiogenic program downstream *Nrf2* activation and to provide anti-apoptotic effects. Furthermore, VEGF secreted by MSCs may synergize with HGF also released by MSCs to protect the endothelial barrier function (Yang, Y. et al. Synergism of MSC-secreted HGF and VEGF in stabilising endothelial barrier function upon lipopolysaccharide stimulation via the Rac1 pathway. Stem Cell Res Ther 6, 250, doi:10.1186/s13287-015-0257-0 (2015)).

In our study, *vegf* was increased upon H₂O₂ exposure in MSC in the PPARβ/δ-agonist primed MSC suggesting that *vegf* was involved in the anti-apoptotic effect of PPARβ/δ and the same profile of expression was observed for HGF. Both HGF and VEGF paracrine factors display anti-apoptotic properties (Kinnaird, T. et al. Marrow-derived stromal cells express genes encoding a broad spectrum of arteriogenic cytokines and promote in vitro and in vivo arteriogenesis through paracrine mechanisms. Circ Res 94, 678-685, doi:10.1161/01.RES.0000118601.37875.AC (2004); Xu M, Uemura R, Dai Y, Wang Y, Pasha Z and Ashraf M. In vitro and in vivo effects of bone marrow stem cells on cardiac structure and function. J Mol Cell Cardiol. 2007;42:441-8). Indeed, VEGF was reported to be secreted by bone marrow derived MSCs leading to anti-apoptotic effects in injured cardiomyocytes via the downregulation of miRNA-23a and miRNA-92 (Song, Y. S. et al. Bone marrow mesenchymal stem cell-derived vascular endothelial growth factor attenuates cardiac apoptosis via regulation of cardiac miRNA-23a and miRNA-92a in a rat model of myocardial infarction. PLoS One 12, e0179972, doi:10.1371/journal.pone.0179972 (2017).

HGF has been described to protect cardiac cells from oxidative stress-induced apoptosis especially by activation of survival pathways like Akt (Wang, Y., Ahmad, N., Wani, M. A. & Ashraf, M. Hepatocyte growth factor prevents ventricular remodeling and dysfunction in mice via Akt pathway and angiogenesis. J Mol Cell Cardiol 37, 1041-1052, doi:10.1016/j.yjmcc.2004.09.004 (2004)). In hypoxic preconditioning MSC resistance to hypoxia-reoxygenation stress was increased and associated to an upregulation of *bcl-2* and *vegf* expression, promoting ERK and Akt (Wang, J. A. et al. Hypoxic preconditioning attenuates hypoxia/reoxygenation-induced apoptosis in mesenchymal stem cells. Acta Pharmacol Sin 29, 74-82, doi:10.1111/j.1745-7254.2008.00716.x (2008)).

Finally, we observed that the significant decreased expression level of *pdgf* observed in MSC exposed to H₂O₂-induced stress was significantly counteracted by pretreating MSC with PPARβ/δ while it tended to be enhanced after a pretreatment with PPARβ/δ antagonist. This is in line with the well-documented role of PDGF, described to protect several cell types from apoptosis, and induce hypertrophic growth (Vantler, M. et al. PDGF-BB protects cardiomyocytes from apoptosis and improves contractile function of engineered heart tissue. J Mol Cell Cardiol 48, 1316-1323, doi:10.1016/j.yjmcc.2010.03.008 (2010)).Regarding cardiomyocytes survival and function, PDGF was shown to improve cardiomyocyte survival, α-sarcomeric actinin content and contractile performance (Vantler, M. et al. PDGF-BB protects cardiomyocytes from apoptosis and improves contractile function of engineered heart tissue. J Mol Cell Cardiol 48, 1316-1323, doi:10.1016/j.yjmcc.2010.03.008 (2010)). Therefore, the enhanced anti-apoptotic effect of MSC primed with PPARβ/δ agonist could be mediated, in part, through the combined increased expression levels of *vegf, hgf* and *pdgf.* This hypothesis should be investigated in loss and gain of function experiments.

In conclusion, our study provides evidence for an augmented MSC-based cell product inducing cardioprotection against IR injury with potent anti-apoptotic effects and an increased number of surviving MSC in the injured myocardium, one hour after the onset of reperfusion.
These results could be of major interest in the clinical setting to improve MSC efficacy for the cardioprotection of injured myocardium in AMI patients.

### Example 3: Effect of the duration of pretreatment of the MSC with a PPARβ/δ agonist

### Materials and Methods

### In vitro:

### Culture of Mesenchymal stem cells

Bone marrow-derived MSCs from C57BL/6 were isolated as previously described (Bouffi C, Bony C, Courties G, Jorgensen C, Noël D. IL-6-dependent PGE2 secretion by mesenchymal stem cells inhibits local inflammation in experimental arthritis. PLoS One. 2010 Dec 7;5(12):e14247). Briefly, bone marrow was flush out from the long bones and the cell suspension (0.5×10⁶cells/cm²) was plated in minimum essential medium (MEM)-α supplemented with 10% fetal bovine serum (FBS), 2 mM glutamine, 100 U/mL penicillin, 100 mg/mL streptomycin and 2 ng/ml human basic fibroblast growth factor (bFGF). At sub-confluence, cells were collected, propagated at a density of 5,000 cells/cm² and used from passage 7.

### Pharmacological preconditioning:

24 hours or 96h before the ex vivo experiment, MSC were incubated with a treatment solution containing the PPARβ/δ agonist diluted in the culture medium (GW0742, 1 µM and G501516, 0.1 µM). The treatment lasted 24 hours or 96 hours. After this preconditioning phase, cells were rinsed twice with PBS, trypsinized with Trypsin EDTA (Invitrogen) during 1 min at 37 °C (5% CO₂. Cells were centrifuged 3 min at 200g and then re-suspended in a preheated Tyrode solution at the final concentration of 5000 cells/ml.

### Ex vivo evaluation:

The cardioprotective effects of preconditioned MSC were evaluated in an ex vivo model of myocardial ischemia-reperfusion. Acute myocardial ischemia-reperfusion was performed in isolated perfused hearts from C57Bl6 mice subjected to 30 minutes of ischemia (no-flow) followed by reperfusion. Male mice were anesthetized with an intraperitoneal injection (IP) of ketamine (14 mg/kg, Imalgène^{®}; Merial), xylazine (14 mg/kg, Rompun^{®}; Bayer) followed by injection of pentobarbital (IP; 76.6 mg/kg; Sanofi-Aventis). Anesthetized mice received 250 U heparin (IP) in order to prevent blood clot formation. After sternotomy, the heart was excised, cannulated through the ascending aorta and quickly mounted on the Langendorff system. A pre-heated Tyrode solution (NaCl 140mM, KCl 5.4 mM, MgCl 1mM, Hepes 5 mM, glucose 5.5 mM, CaCl2 1.8 mM, pH 7.4) was perfused at constant pressure and temperature (37°C).

### Ischemia-reperfusion protocol (IR).

On the Langendorff system, the heart was perfused with the preheated Tyrode solution during 15 minutes (stabilization). Global ischemia was obtained by stopping the perfusion flow (« no-flow ») during 30 minutes. A reperfusion step (60 minutes) was achieved by restoring the flow. The treatment with MSC was applied during reperfusion (MSC in Tyrode solution; 5,000 cells/ml). The control condition (IR) was obtained using only Tyrode.

### Infarct size measurement:

At the end of the IR protocol, the heart was removed from the Langendorff and dissected out. The left ventricle was included in agar (4%), and transversely sliced (1 mm) with a vibratome. To reveal tissue viability, slices were incubated with 2,3,5-triphenyltetrazolium chloride solution (TTC dye) at 37°C for 15 minutes. After a fixation step (4% paraformaldehyde, 48 h), each slice was photographed on each side. Infarct area was quantified by planimetric measurements with ImageJ software and expressed as the percentage of the left ventricle mass.

### Results:

Figure 10 shows that reperfusion with a Tyrode solution containing 5,000 cells/ml (for a total of 4.10⁵ cells/heart) allowed to decrease infarct size by 39,8% compared to the control condition (perfusion with the Tyrode solution, IR).

4 day-preconditioning of MSC with the GW501516 (0.1 µM) allowed to decrease infarct size by 54.5% compared to the control condition (reperfusion with Tyrode solution alone, IR) when perfused at a dose of 4. 10⁵ cells/heart on the Langendorff system.

When the GW501516 pre-treatment was applied during only 24h, MSC (4. 10⁵ cells/heart) were able to decrease infarct size by the same extent leading to a mean value of 37.4 (in % of LV mass) for GW501516 1-day (n=7) compared to 36.05% for GW501516 4 days (n=8) leading to a 52.8 % and a 54.5% -decrease in infarct size, respectively.

Similar results were obtained using GWO742, another PPAR β/δ agonist. Indeed, when MSCs were preconditioned with the GWO742 during 24h (1 µM), infarct size was decreased by 54.6% compared to the non-treated condition (IR).

### Conclusion:

The above data show that pretreating MSC during 24 h with GW501516 provided a pharmacological stimulus leading to the same cardioprotection than that afforded by a 96-hour pretreatment.

A short pretreatment of MSC by a PPARB/δ agonist is thus sufficient to significantly improve the cardioprotective effect of MSCs after myocardial ischemia-reperfusion injury.

Nevertheless our 24-h protocol is superior to the 96-h protocol of WO2015164506 (example 6), with 2 main advantages:
- the duration of pretreatment is reduced by 4 times (1 day versus 4 day),
- the cell dosage is 1000 to 100 000 times less (5,000 cells/ml versus 5-600 x10⁶cells/ml, as described in WO2015164506 example 6).

## Claims

1. An *in vitro* method for preparing a composition comprising mesenchymal stem cells (MSCs) intended for treating ischemia-reperfusion injury, comprising:
a) providing MSCs;
b) cultivating MSCs in a culture medium comprising a PPARB/δ agonist;
c) removing the culture medium and washing the MSCs; and
d) collecting MSCs in a pharmaceutically acceptable carrier, wherein the pharmaceutically acceptable carrier does not comprise a PPARB/δ agonist.

2. The method according to claim 1, wherein the MSCs provided in step a) are bone-marrow derived MSCs or adipose tissue derived MSCs.

3. The method according to claim 1 or claim 2, wherein the MSCs provided in step a) are allogenic and have been obtained from a healthy subject.

4. The method according to any one of claims 1 to 3, wherein the duration of step b) is comprised between 12 hours and 72 hours, preferably between 18 hours and 48 hours, more preferably between 20 hours and 30 hours, such as 24 hours.

5. The method according to any one of claims 1 to 4, wherein the concentration of the PPARB/δ agonist in the culture medium of step b) is comprised between 0.01 µM and 10 µM, preferably between 0.05 µM and 5 µM, more preferably between 0.1 µM and 1 µM.

6. The method according to any one of claims 1 to 5, wherein the PPARB/δ agonist is selected from GW0742 and GW501516.

7. The method according to any one of claims 1 to 6, wherein the culture medium of step b) does not contain a viral vector for transducing MSCs.

8. The method according to any one of claims 1 to 7, wherein the washing in step c) is performed with a solution that does not comprise a PPARB/δ agonist.

9. The method according to any one of claims 1 to 8, wherein in step d), MSCs are collected in a pharmaceutically acceptable carrier at a concentration of 10² to 10⁸ MSC cells/mL, preferably at a concentration of 10³ to 10⁷ MSC cells/mL.

10. A composition comprising PPARB/δ-primed mesenchymal stem cells (MSCs), wherein said composition has been obtained by the *in vitro* method according to any one of claims 1 to 9.

11. A composition according to claim 10, for use in a method of treatment, wherein the method of treatment does not comprise administering a PPARB/δ agonist to the subject.

12. A composition according to claim 10, for use in a method of treatment of ischemia-reperfusion injury, preferably myocardial or kidney ischemia-reperfusion injury, in a subject in need thereof, wherein the method of treatment does not comprise administering a PPARB/δ agonist to the subject.

13. The composition for use according to claim 12, wherein said composition is administered to the subject by intravenous or intracoronary administration.

14. The composition for use according to claim 12 or claim 13, wherein the total amount of MSCs administered to the subject is comprised between 5x10⁶ and 5x10¹⁰ PPARB/δ-primed MSCs.

15. The composition for use according to any one of claims 12 to 14, wherein the composition is administered within 7 days of the onset of ischemia-reperfusion injury or during reperfusion of the ischemic organ.
